(19) Europäisches Patentamt
European Patent Office
Office européen des brevets

(11) Veröffentlichungsnummer : **0 059 386**
**B1**

(12) # EUROPÄISCHE PATENTSCHRIFT

(45) Veröffentlichungstag der Patentschrift : **18.09.85**

(21) Anmeldenummer : **82101268.9**

(22) Anmeldetag : **19.02.82**

(51) Int. Cl.[4] : **C 07 D487/04**, C 07 D487/14, A 61 K 31/55 // (C07D487/04, 243:00, 235:00),(C07D487/04, 243:00, 209:00)

(54) **Imidazodiazepine, Zwischenprodukte und Verfahren zu ihrer Herstellung, sowie diese enthaltende Arzneimittel.**

(30) Priorität : **27.02.81 CH 1338/81**

(43) Veröffentlichungstag der Anmeldung : **08.09.82 Patentblatt 82/36**

(45) Bekanntmachung des Hinweises auf die Patenterteilung : **18.09.85 Patentblatt 85/38**

(84) Benannte Vertragsstaaten : **BE CH DE FR GB IT LI NL SE**

(56) Entgegenhaltungen :
**EP-A- 0 027 214**
**DE-A- 2 609 486**
**DE-A- 2 813 549**
**FR-A- 2 248 050**
**US-A- 4 022 767**

(73) Patentinhaber : **F. HOFFMANN-LA ROCHE & CO. Aktiengesellschaft**
**CH-4002 Basel (CH)**

(72) Erfinder : **Hunkeler, Walter, Dr.**
**Im Stigler 32**
**CH-4465 Magden (CH)**
Erfinder : **Kyburz, Emilio, Dr.**
**Unterer Rebbergweg 127**
**CH-4153 Reinach (CH)**

(74) Vertreter : **Lederer, Franz, Dr. et al**
**Patentanwälte Dr. Lederer Franz Meyer-Roxlau Reiner F. Lucile-Grahn-Strasse 22**
**D-8000 München 80 (DE)**

**EP 0 059 386 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Imidazodiazepine. Im speziellen betrifft sie Imidazodiazepine der allgemein Formel

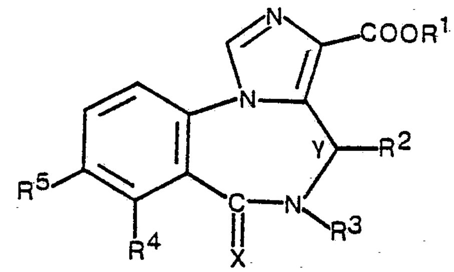

(I)

worin $R^1$ Methyl, Aethyl oder Isopropyl, einer der Reste $R^4$ und $R^5$ Wasserstoff und der andere Nitro oder Cyano bedeuten, und entweder $R^2$ Wasserstoff und $R^3$ Wasserstoff oder $C_1$-$C_7$ Alkyl bedeuten, oder $R^2$ und $R^3$ zusammen Dimethylen, Trimethylen oder Propenylen bedeuten und das mit $\gamma$ bezeichnete Kohlenstoffatom die (S)- oder (R,S)-Konfiguration aufweist, und X ein Sauerstoff- oder Schwefelatom bedeutet, und pharmazeutisch annehmbare Säureadditionssalze davon.

Diese Verbindungen sind neu ; sie zeichnen sich durch wertvolle pharmakodynamische Eigenschaften aus und können demnach zur Bekämpfung bzw. Verhütung von Krankheiten verwendet werden.

Gegenstand der vorliegenden Erfindung sind Verbindungen der allgemeinen Formel I und pharmazeutisch annehmbare Säureadditionssalze davon als solche und als pharmazeutische Wirkstoffe, die Herstellung dieser Verbindungen und Zwischenprodukte für die Herstellung dieser Verbindungen, ferner Arzneimittel, enthaltend eine Verbindung der allgemeinen Formel I oder ein pharmazeutisch annehmbares Säureadditionssalz davon.

Der Ausdruck « $C_1$-$C_7$ Alkyl » bezeichnet gesättigte Kohlenwasserstoffreste, welche geradkettig oder verzweigt sein können, mit höchstens 7, vorzugsweise höchstens 4 Kohlenstoffatomen, wie Methyl, Aethyl, n-Propyl, Isopropyl, n-Butyl, s-Butyl, t-Butyl und dergleichen.

Wenn $R^2$ Wasserstoff bedeutet, so ist die bevorzugte Bedeutungsmöglichkeit von $R^3$ Methyl. Wenn $R^2$ und $R^3$ zusammen Dimethylen oder Trimethylen bedeuten, so weist das in Formel I mit $\gamma$ bezeichnete Kohlenstoffatom vorzugsweise die (S)-Konfiguration auf.

Ganz besonders bevorzugte Verbindungen im Rahmen der vorliegenden Erfindung sind Aethyl-5,6-dihydro-5-methyl-7-nitro-6-oxo-4H-imidazo [1,5-a] [1,4] benzodiazepin-3-carboxylat und Aethyl-7-cyano-5,6-dihydro-5-methyl-6-oxo-4H-imidazo [1,5-a] [1,4] benzodiazepin-3-carboxylat.

Weitere im Rahmen der vorliegenden Erfindung bevorzugte von der allgemeinen Formel I umfasste Verbindungen sind :

Aethyl-5,6-dihydro-5-methyl-8-nitro-6-nitro-6-oxo-4H-imidazo-[1,5-a] [1,4] benzodiazepin-3-carboxylat,

Aethyl-8-cyano-5,6-dihydro-5-methyl-6-oxo-4H-imidazo- [1,5-a] [1,4] benzodiazepin-3-carboxylat und

Aethyl-(S)-(+)-11,12,13,13a-tetrahydro-7-nitro-9-oxo-9H-imidazo [1,5-a] pyrrolo [2,1-c] [1,4] benzodiazepin-1-carboxylat.

Die Imidazodiazepine der allgemeinen Formel I und ihre pharmazeutisch annehmbaren Säureadditionssalze können erfindungsgemäss hergestellt werden, indem man

a) eine Verbindung der allgemeinen Formel

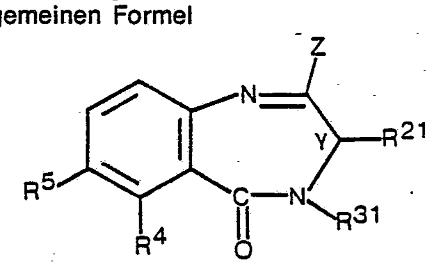

(II)

worin $R^4$ und $R^5$ obige Bedeutung besitzen und Z eine Abgangsgruppe bedeutet, und entweder $R^{21}$ Wasserstoff und $R^{31}$ $C_1$-$C_7$ Alkyl bedeuten, oder $R^{21}$ und $R^{31}$ zusammen Dimethylen, Trimethylen oder Propenylen bedeuten und das mit $\gamma$ bezeichnete Kohlenstoffatom die (S)- oder (R,S)-Konfiguration aufweist, in Gegenwart einer Base mit einem Isocyanessigester der allgemeinen Formel

$$CN—CH_2—COOR^1 \qquad (III)$$

worin $R^1$ obige Bedeutung besitzt, umsetzt, oder

b) in einer Verbindung der allgemeinen Formel

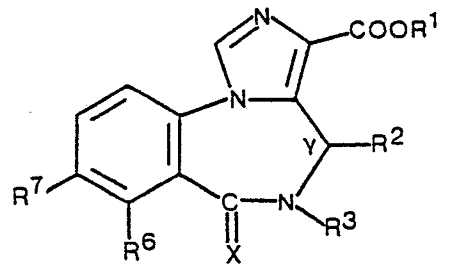

(IV)

worin einer der Reste $R^6$ und $R^7$ Wasserstoff und der andere Halogen bedeutet, und $R^1$, $R^2$, $R^3$ und X obige Bedeutung besitzen, das Halogenatom durch die Cyanogruppe ersetzt, oder

c) eine Verbindung der allgemeinen Formel

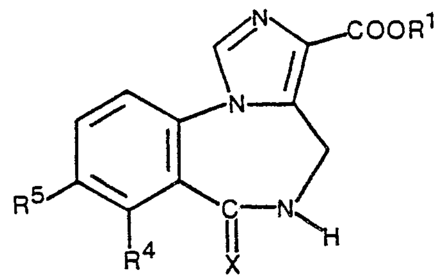

(Ia)

worin $R^1$, $R^4$, $R^5$ und X obige Bedeutung besitzen, an der sekundären Aminogruppe entsprechend substituiert, oder

d) in einer Verbindung der allgemeinen Formel

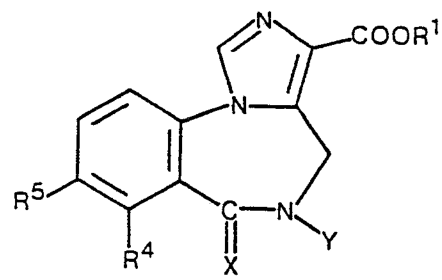

(V)

worin $R^1$, $R^4$, $R^5$ und X obige Bedeutung besitzen, und Y eine schutzgruppe bedeutet, die Schutzgruppe abspaltet, oder

e) eine Verbindung der allgemeinen Formel

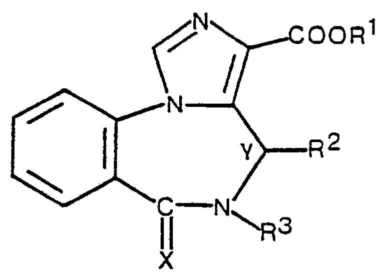

(VI)

worin $R^1$, $R^2$, $R^3$ und X obige Bedeutung besitzen, nitriert, oder

f) eine Verbindung der allgemeinen Formel

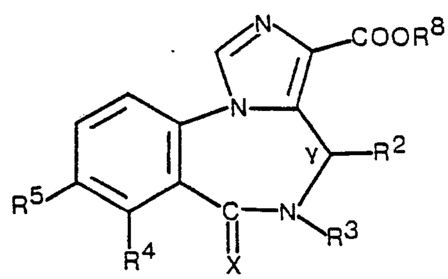

(VII)

worin $R^2$, $R^3$, $R^4$, $R^5$ und X obige Bedeutung besitzen, und $R^8$ $C_1$-$C_7$ Alkyl bedeutet, umestert, oder

g) in einer Verbindung der allgemeinen Formel

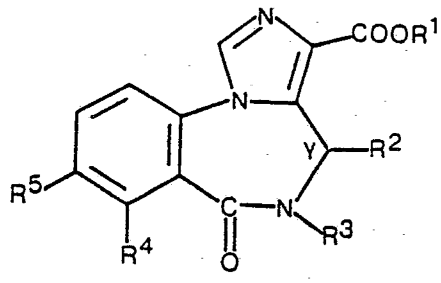

(Ib)

worin $R^1$, $R^2$, $R^3$, $R^4$ und $R^5$ obige Bedeutung besitzen, die Carbonyl-Gruppe in die Thiocarbonyl-Gruppe überführt, und

h) erwünschtenfalls eine Verbindung der allgemeinen Formel I in ein pharmazeutisch Säureadditionssalz überführt.

Gemäss Verfahrensvariante a) können Verbindungen der allgemeinen Formel I demnach aus Verbindungen der allgemeinen Formel II und Isocyanessigestern der allgemeinen Formel III hergestellt werden. Die in Formel II mit Z bezeichnete Abgangsgruppe ist beispielsweise eine leicht abspaltbare Phosphinylgruppe, z. B. eine Gruppe der Formel

$$-O\overset{O}{\overset{\|}{P}}(OR^9)_2 \text{ oder } -O\overset{O}{\overset{\|}{P}}(NRR')_2$$

worin $R^9$ niederes Alkyl und R und R' je niederes Alkyl, Allyl, Phenyl oder substituiertes Phenyl oder zusammen mit dem Stickstoffatom einen unsubstituierten oder substituierten heterocyclischen Ring mit 3-8 Gliedern (wie Morpholin) bedeuten, ein Halogenatom, eine Alkylthiogruppe, eine Aralkylthiogruppe, eine N-Nitrosoalkylaminogruppe, eine Alkoxygruppe, eine Mercaptogruppe und dergleichen (wenn Z eine Mercaptogruppe bedeutet, so handelt es sich bei der entsprechenden Verbindung der Formel II um die Iminothiol-Form des entsprechenden Thiolactams). Die Umsetzung der Verbindungen der Formeln II und III erfolgt in einem inerten Lösungsmittel, wie Dimethylformamid, Hexamethylphosphorsäuretriamid, Dimethylsulfoxid, Tetrahydrofuran oder irgend einem anderen geeigneten organischen Lösungsmittel und in Gegenwart einer Base, die genügend stark basisch ist, um das Anion des Isocyanessigesters der Formel III zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperatur liegt zweckmässigerweise zwischen etwa − 40 °C und etwa Raumtemperatur.

Gemäss Verfahrensvariante b) kann eine Verbindung der allgemeinen Formel I, worin einer der Reste $R^4$ und $R^5$ Cyano und der andere Wasserstoff bedeutet, dadurch hergestellt werden, dass man in einer Verbindung der allgemeinen Formel IV das Halogenatom durch die Cyanogruppe ersetzt. Diese Reaktion kann nach an sich bekannten und jedem Fachmann geläufigen Methoden durchgeführt werden, beispielsweise durch Behandeln eines Halogenids der allgemeinen Formel IV, vorzugsweise ein Bromid oder Jodid, mit Kupfer (I) cyanid in einem inerten organischen Lösungsmittel. Geeignete Lösungsmittel sind z. B. hochsiedende Lösungsmittel, wie Pyridin, Dimethylformamid, N-Methylpyrrolidinon und dergleichen. Die Reaktion kann je nach verwendetem Lösungsmittel und Halogenid in einem Temperaturbereich von etwa Raumtemperatur bis etwa 200 °C durchgeführt werden.

Gemäss Verfahrensvariante c) können Verbindungen der allgemeinen Formel I dadurch hergestellt werden, dass man Verbindungen der Formel Ia an der sekundären Aminogruppe in 5-Stellung entsprechend substituiert. Diese Substitution erfolgt nach an sich bekannten Methoden unter Verwendung eines den gewünschten $C_1$-$C_7$ Alkylrest abgebenden Mittels, beispielsweise entsprechende organische Sulfonsäure-Alkylester (z. B. p-Toluolsulfonsäure-methylester), entsprechende Dialkylsulfate, wie Dimethylsulfat und Diäthylsulfat, entsprechende Alkylshalogenide, wie Methyljodid, Aethyljodid oder Aethylbromid, und dergleichen. Die Verbindung der allgemeinen Formel Ia wird dabei zweckmässigerweise in Form eines Alkalimetall-Salzes eingesetzt ; man erreicht dies zweckmässigerweise dadurch, dass man die Reaktion in Gegenwart einer starken Base ablaufen lässt oder die Verbindung der Formel Ia vor der Reaktion mit dem Alkylierungsmittel in ein Alkalimetallsalz überführt. Geeignete Basen sind Alkalimetall-alkoxide, wie Natriummethoxid oder Kalium-t-butoxid, Alkalimetall-hydride, wie Natriumhydrid, Alkalimetall-amide, wie Lithiumamid oder Lithiumdiisopropylamid, und dergleichen. Die Reaktion wird zweckmässigerweise in Gegenwart eines inerten organischen Lösungsmittels durchgeführt. Hierfür eignen sich z. B. Dimethylformamid, Dimethylsulfoxid, Essigester, niedere Alkanole und dergleichen ; viele andere Lösungsmittel und auch Lösungsmittelgemische sind ebenfalls geeignet und

ihre Auswahl bietet dem Fachmann keinerlei Schwierigkeiten. Die Reaktionstemperatur ist in ziemlich weiten Grenzen varierbar und wird in der Regel zwischen etwa Raumtemperatur und etwa der Siedetemperatur des Reaktionsgemisches liegen.

Gemäss Verfahrensvariante d) können Verbindungen der allgemeinen Formel I hergestellt werden, indem man in Verbindungen der allgemeinen Formel V die mit Y bezeichnete Schutzgruppe abspaltet. Es kommen dabei nur Schutzgruppen in Frage, die unter milden sauren Bedingungen abgespalten werden können, beispielsweise mit verdünnten wässrigen Mineralsäuren, wie verdünnte Salzsäure oder verdünnte Schwefelsäure, Trifluoressigsäure oder dergleichen, gegebenenfalls unter Zusatz eines Lösungsvermittlers, wie Tetrahydrofuran, Dioxan, Essigsäure, N,N-Dimethylformamid oder dergleichen. Zweckmässigerweise arbeitet man bei Temperaturen von etwa Raumtemperatur bie Siedetemperatur der Reaktionsmischung, wobei letzteres bevorzugt wird. Eine besonders geeignete Schutzgruppe ist die 2,4-Dimethoxybenzyl-Gruppe, die zweckmässigerweise mit Trifluoressigsäure abgespalten wird, vorzugsweise bei der Siedetemperatur des Reaktionsgemisches.

Gemäss Verfahrensvariante e) können Verbindungen der allgemeinen Formel I, worin $R^4$ Wasserstoff und $R^5$ Nitro bedeuten, dadurch hergestellt werden, dass man eine Verbindung der allgemeinen Formel VI nitriert. Die Nitrierung kann nach an sich bekannten und jedem Fachmann geläufigen Methoden durchgeführt werden. Je nach Reaktivität der Verbindung der Formel VI kann die Nietrierung mit konzentrierter Salpetersäure oder mit einem Gemisch von konzentrierter Salpetersäure und konzentrierter Schwefelsäure in einem Temperaturbereich von etwa 0 °C bis etwa 150 °C durchgeführt werden.

Gemäss Verfahrensvariante f) können Verbindungen der allgemeinen Formel I durch Umesterung von Verbindungen der allgemeinen Formel VII hergestellt werden, d. h. dass in Verbindungen der allgemeinen Formel VII der mit $R^8$ bezeichnete Alkylrest gegen einen Rest $R^1$ ausgetauscht wird, wobei natürlich $R^8$ und $R^1$ jeweils verschieden sind. Sofern $R^8$ in Formel VII Methyl, Aethyl oder Isopropyl bedeutet, fallen die Verbindungen der Formel VII unter die eingangs definierte Formel I. $R^8$ kann jedoch natürlich auch eine andere $C_1$-$C_7$ Alkylgruppe bedeuten.

Diese Umesterung erfolgt in an sich bekannter Weise durch Umsetzen einer Verbindung der allgemeinen Formel VII mit einem dem gewünschten Rest $R^1$ entsprechenden Alkohol, d. h. Methanol bzw. Aethanol bzw. Isopropanol, bei Raumtemperatur oder unter Erwärmen auf eine Temperatur von etwa 25° bis 150 °C. Vorzugsweise wird die Umesterung in Gegenwart einer Base durchgeführt, wobei sich im vorliegenden Fall insbesondere Kaliumcyanid oder ähnliche schwache Basen oder das dem gewünschten Rest $R^1$ entsprechende Alkoholat eignen. Als Lösungsmittel dient vorzugsweise der dem Rest $R^1$ im gewünschten Endprodukt der Formel I, entsprechende Alkohol ; die Umesterung kann jedoch auch in einem inerten organischen Lösungsmittel erfolgen, beispielsweise in einem aromatischen Kohlenwasserstoff, wie Benzol oder Xylol, einem Aether, wie Dioxan, Tetrahydrofuran oder Aethylenglykol-dimethyläther, in Dimethylformamid, in Dimethylsulfoxid oder dergleichen. Bei dieser Umesterung kann man nicht nur einen niedriger siedenden Alkohol durch einen höher siedenden Alkohol, sondern auch einen höher siedenden Alkohol durch einen niedriger siedenden Alkohol ersetzen.

Die Umesterung kann indessen ohne weiteres auch mehrstufig durchgeführt werden, beispielsweise dadurch, dass man die eingesetzte Verbindung der Formel VII zur entsprechenden freien Carbonsäure hydrolysiert, aus dieser ein reaktionsfähiges funktionelles Derivat (z. B. ein Säurechlorid oder dergleichen) herstellt und anschliessend dieses reaktionsfähige Carbonsäurederivat mit dem der Bedeutung von $R^1$ in der gewünschten Verbindung der Formel I entsprechenden Alkohol zur Reaktion bringt.

Gemäss Verfahrensvariante g) können Verbindungen der allgemeinen Formel Ib in entsprechende Verbindungen der Formel I, worin X ein Schwefelatom bedeutet, übergeführt werden, und zwar durch Behandeln mit einem Schwefelungsmittel, was in an sich bekannter Weise erfolgen kann. Beispielsweise kann man als Schwefelungsmittel Phosphorpentasulfid verwenden, wobei dieses vorzugsweise im Ueberschuss eingesetzt wird und die Reaktion vorteilhafterweise in einem inerten organischen Lösungsmittel, wie Dioxan, Methylenchlorid oder dergleichen, in Gegenwart von Triäthylamin bei einer Temperatur von etwa 50 °C bis zur Rückflusstemperatur des Reaktionsgemisches erfolgt. Als Schwefelungsmittel eignen sich indessen auch Verbindungen wie 2,4-Bis(p-methoxyphenyl)-1,3,2,4-dithiaphosphetan-2,4-disulfid ; derartige Schwefelungsmittel werden ungefähr in der berechneten Menge eingesetzt, und die Reaktion erfolgt in Gegenwart eines inerten Lösungsmittels, wie Toluol und Xylol, zweckmässigerweise bei Rückflusstemperatur des Reaktionsgemisches oder in Hexamethyl-phosphorsäuretriamid zwischen etwa 60 und 110 °C.

Gemäss Verfahrensvariante h) können Verbindungen der allgemeinen Formel I erfindungsgemäss in pharmazeutisch annehmbare Säureadditionssalze übergeführt werden. Die Herstellung von solchen pharmazeutisch annehmbaren Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluol-sulfonate, Oxalate und dergleichen.

Die als Ausgangsstoffe verwendeten Verbindungen der allgemeinen Formel II können ausgehend von Verbindungen der Formel

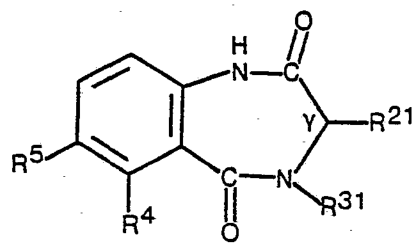

(VIII)

worin $R^{21}$, $R^{31}$, $R^4$ und $R^5$ obige Bedeutung besitzen, hergestellt werden, und zwar nach Methoden, welche an sich bekannt sind, vgl. z. B. die Belgischen Patentschriften No. 802 233, 833 249 und 865 653, die Amerikanische Patentschrift No 3 681 341 und J. Org. Chemistry *29,* 231 (1964).

Verschiedene der weiter unten folgenden Beispiele enthalten detaillierte Angaben betreffend die Herstellung von Verbindungen der allgemeinen Formel II aus Verbindungen der allgemeinen Formel VIII.

Die Verbindungen der Formel VIII ihrerseits gehören einer an sich bekannten Stoffklasse an und können in Analogie zu den bekannten Vertretern dieser Stoffklasse von jedem Fachmann leicht hergestellt werden. So können die Verbindungen der Formel VIII beispielsweise durch Umsetzen eines entsprechenden Carbonsäureanhydrids der allgemeinen Formel

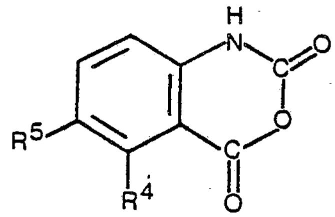

(IX)

worin $R^4$ und $R^5$ obige Bedeutung besitzen, mit einer Aminosäure der allgemeinen Formel

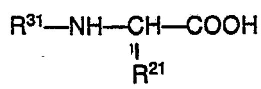

(X)

worin $R^{21}$ und $R^{31}$ obige Bedeutung besitzen, hergestellt werden.

Verbindungen der allgemeinen Formel VIII, worin $R^{21}$ Wasserstoff und $R^{31}$ $C_1$-$C_7$ Alkyl bedeuten, können aber auch ausgehend von Verbindungen der allgemeinen Formel

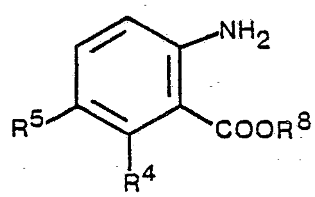

(XI)

worin $R^4$, $R^5$ und $R^8$ obige Bedeutung besitzen, hergestellt werden, beispielsweise durch Behandeln einer solchen Verbindungen mit einem reaktiven Derivat einer α-Halogenessigsäure, z. B. α-Chloressigsäurechlorid, und Umsetzen des erhaltenen Zwischenproduktes mit einem $C_1$-$C_7$ Alkylamin, wie Methylamin, Aethylamin oder dergleichen. Man erhält somit Verbindungen der allgemeinen Formel

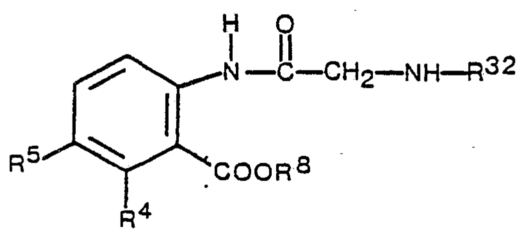

(XII)

worin $R^4$, $R^5$ und $R^8$ obige Bedeutung besitzen, und $R^{32}$ $C_1$-$C_7$ Alkyl bedeuten.

Durch Cyclisieren von Verbindungen der allgemeinen Formel XII gelangt man zu Verbindungen der allgemeinen Formel VIII, worin $R^{21}$ Wasserstoff und $R^{31}$ $C_1$-$C_7$ Alkyl bedeuten. Diese Cyclisierung erfolgt

beispielsweise durch kurzzeitiges Erhitzen einer entsprechenden Verbindung der allgemeinen Formel XII auf Temperaturen von etwa 100° bis etwa 300 °C.

Es ist auch möglich eine Verbindung der Formel XI mit einem reaktiven Derivat einer Carbonsäure der allgemeinen Formel

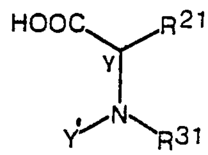

(XIII)

worin $R^{21}$ und $R^{31}$ obige Bedeutung besitzen und Y′ eine Schutzgruppe bedeutet, beispielsweise einem Carbonsäurechlorid oder dergleichen, umzusetzen. Nach Entfernen der mit Y′ bezeichneten Schutzgruppe aus einer so erhaltenen Verbindung der allgemeinen Formel

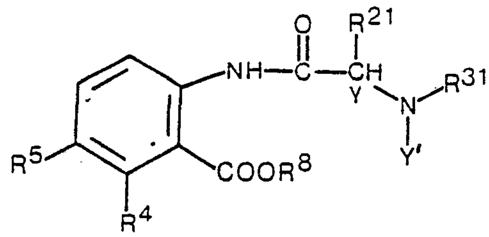

(XIV)

worin $R^{21}$, $R^{31}$, $R^4$, $R^5$, $R^8$ und Y′ obige Bedeutung besitzen, und Cyclisieren, in Analogie zur Herstellung von Verbindungen der Formel VIII aus solchen der Formel XII, erhält man eine Verbindung der allgemeinen Formel VIII.

Um eine Verbindung der allgemeinen Formel

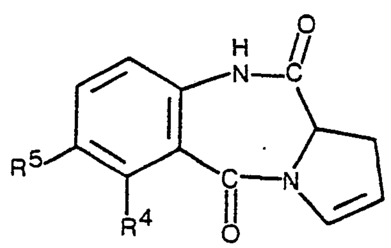

(VIIIa)

worin $R^4$ und $R^5$ obige Bedeutung besitzen, zu erhalten, kann man auch in an sich bekannter Weise in einer Verbindung der allgemeinen Formel

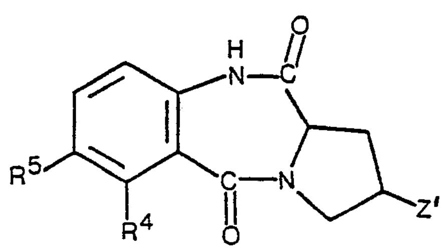

(XV)

worin $R^4$ und $R^5$ obige Bedeutung besitzen, und Z′ eine Abgangsgruppe bedeutet, die mit Z′ bezeichneten Abgangsgruppe eliminieren. Als Abgangsgruppen kommen beispielsweise Sulfonsäuregruppen, wie Methansulfonyloxy, p-Toluolsulfonyloxy oder dergleichen, Halogenatome, wie Chlor, Brom und Jod, oder dergleichen, in Frage. Die Abspaltung erfolgt mit einer Base, wie Natriumhydrid, in einem inerten organischen Lösungsmittel, wie Dimethylformamid.

Verbindungen der Formel XV können in Analogie zur Herstellung von Verbindungen der allgemeinen Formel VIII aus Verbindungen der allgemeinen Formel IX und X oder aus solchen der Formel XIV hergestellt werden.

Verbindungen der Formel VIII, worin einer der Reste $R^4$ und $R^5$ Wasserstoff und der andere Cyano bedeutet, können aber auch hergestellt werden, indem man in einer Verbindung der allgemeinen Formel

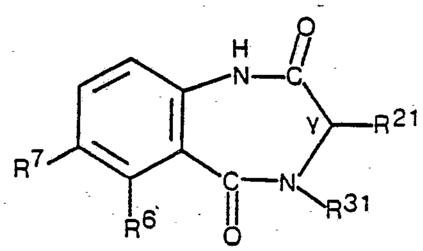

(XVI)

worin $R^{21}$, $R^{31}$, $R^6$ und $R^7$ obige Bedeutung besitzen, das Halogenatom durch die Cyanogruppe ersetzt, und zwar in Analogie zur weiter oben beschriebenen Verfahrensvariante b). Verbindungen der Formel VIII, worin $R^4$ Wasserstoff und $R^5$ Nitro bedeutet, können auch hergestellt werden, indem man eine Verbindung der allgemeinen Formel

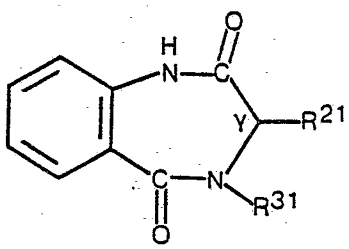

(XVII)

worin $R^{21}$ und $R^{31}$ obige Bedeutung besitzen, nitriert, und zwar in Analogie zur weiter oben beschriebenen Verfahrensvariante e). Die Verbindungen der Formeln XVI und XVII sind bekannt oder nach an sich bekannten Methoden leicht herstellbar; vgl. die weiter oben zur Herstellung von Verbindungen der Formel VIII aus solchen der Formeln IX und X, XII, XIV bzw. XV beschriebenen Methoden.

Die als Ausgangsstoffe verwendeten Verbindungen der allgemeinen Formeln IV und VI können in Analogie zu den weiter oben beschriebenen Verfahren zur Herstellung von Verbindungen der allgemeinen Formel I hergestellt werden; beispielsweise in Analogie zu Verfahrensvariante a), ausgehend von Verbindungen der Formel XVI bzw. XVII, oder in Analogie zu Verfahrensvarianten c), d), f) oder g) und zu den für die Herstellung der entsprechenden Ausgangsstoffe beschriebenen Methoden.

Verbindungen der Formel V können ausgehend von Verbindungen der allgemeinen Formel

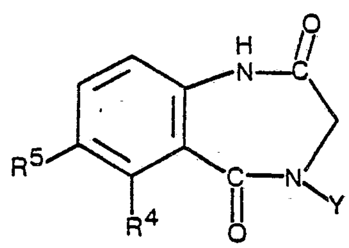

(XVIII)

worin $R^4$, $R^5$ und Y obige Bedeutung besitzen, in Analogie zu den weiter oben beschriebenen Methoden zur Herstellung von Verbindungen der allgemeinen Formel I, worin $R^2$ Wasserstoff und $R^3$ $C_1$-$C_7$ Alkyl bedeuten, hergestellt werden, und zwar in Analogie zu Verfahrensvarianten a), b), f) und g) und zu den für die Herstellung der entsprechenden Ausgangsstoffe beschriebenen Methoden.

Verbindungen der allgemeinen Formel VII, worin $R^8$ nicht Methyl, Aethyl und Isopropyl bedeutet können in Analogie zu den weiter oben beschriebenen Methoden zur Herstellung von Verbindungen der allgemeinen Formel I, worin $R^1$ Methyl, Aethyl oder Isopropyl bedeutet, hergestellt werden; und zwar in Analogie zu Verfahrensvarianten a), b), c), d), e), f) und g) und zu den für die Herstellung der entsprechenden Ausgangsstoffe beschriebenen Methoden; sie sind neu und ebenfalls Gegenstand der vorliegenden Erfindung.

Auch die als Ausgangsstoffe verwendeten Verbindungen der allgemeinen Formel V sind neu und ebenfalls Gegenstand der vorliegenden Erfindung.

Gewisse Verbindungen der Formel II, worin $R^{21}$ Wasserstoff und $R^{31}$ $C_1$-$C_7$ Alkyl und einer der Reste $R^4$ und $R^5$ Wasserstoff und der andere Nitro bedeutet, sind in US-A-4 022 767 beschrieben. Neu und ebenfalls Gegenstand der vorliegenden Erfindung sind jedoch alle jene Verbindungen der Formel II, worin $R^4$ bzw. $R^5$ nicht Nitro bedeutet, wenn $R^{21}$ Wasserstoff und $R^{31}$ $C_1$-$C_7$ Alkyl bedeuten.

Wie eingangs erwähnt, sind die Verbindungen der allgemeinen Formel I neu und besitzen äusserst wertvolle pharmakodynamische Eigenschaften. Sie weisen nur eine geringe Toxizität auf, und es hat sich gezeigt, dass sie eine ausgeprägte Affinität zu den zentralen Benzodiazepin-Rezeptoren haben. Aus De-A-2 609 486 und DE-A-2 813 549 sind 6-Aryl-4H-imidazo [1,5-a] [1,4] benzodiazepin-3-carboxylate bekannt, welche muskelrelaxierende, sedative, anticonvulsive und anxiolytische Eigenschaften aufweisen, wogegen die Verbindungen der allgemeinen Formel I die zentral dämpfenden, muskelrelaxierenden, ataktischen, blutdrucksenkenden und atemdepressiven Eigenschaften von tranquilisierend wirksamen 1,4-Benzodiazepinen zu antagonisieren vermögen.

Die Affinität von Verbindungen der allgemeinen Formel I zu den zentralen Benzodiazepin-Rezeptoren wurde nach der in Life Science *20,* 2101-2110 (1977) und Science *198,* 849-851 (1977) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ (« 50 % inhibiting concentration ») diejenige Konzentration der jeweiligen Testsubstanz, welche eine 50-proz. Hemmung der spezifischen Bindung des Tritiierten Diazepams an die spezifischen Benzodiazepin-Rezeptoren im cerebralen Cortex bewirkt.

Eine der typischen Eigenschaften von tranquilisierend wirksamen 1,4-Benzodiazepinen in versuchstieren ist ihre ausgeprägte anticonvulsive Wirkung, welche beispielsweise im bekannten und allgemein anerkannten Pentetrazol-Test nachgewiesen werden kann. Diese Eigenschaft wurde verwendet, um den nachstehend beschriebenen Test auszuarbeiten, der es erlaubt, Verbindungen zu ermitteln, die die zentralen Eigenschaften von tranquilisierend wirksamen 1,4-Benzodiazepinen zu antagonisieren vermögen.

In diesem Test verabreicht man Mäusen eine Stunde vor dem Pentetrazol (120 mg/kg, i.p.) 5 mg/kg (i. p.) Diazepam (d.h. eine supramaximale Dosis, die im Pentetrazol-Test an mehr als 900 Mäusen alle Versuchstiere vor krampfartigen anfällen schützte) und 15 Minuten vor dem Pentetrazol die zu testende Verbindung p.o... Die antagonistische Wirksamkeit der untersuchten Verbindungen, d.h. ihr Vermögen, die Wirkung des Diazepams im Pentetrazol-Test aufzuheben, wird ermittelt, indem man die Mäuse auszählt, die in diesem Test krampfartige Anfälle erleiden.

In der nachstehenden Tabelle werden die Resultate dargestellt, welche mit repräsentativen Vertretern der durch die allgemeinen Formel I definierten Verbindungsklasse in dem vorstehend geschilderten Versuch erhalten worden sind. Angegeben wird für jede der darin figurierenden Verbindung der $ED_{50}$-Wert. Als $ED_{50}$ bezeichnet man die Menge der jeweiligen Testverbindung in mg/kg (p. o.), die bei 50 % der Tiere den Diazepam-Effekt in obigem Versuch aufhebt. Ausserdem enthält die Tabelle die oben definierten $IC_{50}$-Werte für alle darin angegebenen Testverbindungen.

(Siehe Tabelle Seite 10 f.)

Tabelle

| Verbindung der Formel I, worin | | | | | | | $IC_{50}$ in nM/l | $ED_{50}$ in mg/kg p.o. |
|---|---|---|---|---|---|---|---|---|
| $R^1$ | $R^2$ | $R^3$ | Konfiguration | $R^4$ | $R^5$ | X | | |
| $-CH_2CH_3$ | H | $-CH_3$ | - | $-NO_2$ | H | O | 1,6 | 0,8 |
| $-CH_2CH_3$ | H | $-CH_3$ | - | H | $-NO_2$ | O | 20,0 | 3,0 |
| $-CH_2CH_3$ | H | $-CH_3$ | - | H | -CN | O | 11,0 | 16,0 |
| $-CH_2CH_3$ | H | $-CH_3$ | - | -CN | H | O | 1,3 | 1,12 |
| $-CH_2CH_3$ | $-(CH_2)_3-$ | | (S) | H | $-NO_2$ | O | 90,0 | 12,0 |

Wie bereits erwähnt, antagonisieren die Verbindungen der Formel I die zentral dämpfenden, muskelrelaxierenden, ataktischen, blutdrucksenkenden und atemdepressiven Eigenschaften von tranquilisierend wirksamen 1,4-Benzodiazepinen. Letztere stehen in der Therapie in vielfältigem Gebrauch und werden oft is hohen Dosierungen angewandt, sodass die oben erwähnten Wirkungen auch als Nebenwirkungen stark hervortreten können. Die Verbindungen der Formel I können bei Intoxikationen, bei welchen übermässige Einnahme von tranquilisierend wirksamen 1,4-Benzodiazepinen beteiligt ist, als Antidot verwendet werden. Sie eignen sich auch zur Abkürzung einer durch tranquilisierend wirksame 1,4-Benzodiazepine eingeleiteten Anästhesie in der Chirurgie und in der Geburtshilfe. Bei Neugeborenen kann eine eventuelle Atemdepression, die auf die Gabe von tranquilisierend wirksamen 1,4-Benzodiazepinen an die Mutter zurückgeht, aufgehoben werden. Die Verbindungen der Formel I können auch dazu verwendet werden, bei 1,4-Benzodiazepinen die in anderen Indikationsgebieten eingesetzt werden, die in einem solchen Fall unerwünschten Wirkungen auf das zentrale Nervensystem zu unterdrücken. Beispiele derartiger, in anderen Indikationsgebieten einsetzbarer 1,4-Benzodiazepine sind die in den Britischen Patenschriften n° 1 444 529 und 1 474 305 beschriebenen schistosomiazid wirksamen 1,4-Benzodiazepine, wie das (+)-5-(o-Chlorphenyl)-1,3-dihydro-3-methyl-7-nitro-2H-1,4-benzodiazepin-2-on.

Die Verbindungen der allgemeinen Formel I und ihre pharmazeutisch annehmbaren Säureadditionsalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von Tabletten, Lacktabletten, Dragées und Hartgelatinekapseln können die Verfahrensprodukte mit pharmazeutisch inerten, anorganischen oder organischen Trägern verarbeitet werden. Als solche Träger kann man für Tabletten, Dragées und Hartgelatinekapseln z. B. Lactose, Maisstärke oder Derivate davon, Talk, Stearinsäure oder deren Salze etc. verwenden. Für Weichgelatinekapseln eignen sich als Träger z. B. pflanzliche Oele, Wachse, Fette, halbfeste und flüssige Polyole etc. Zur Herstellung von Lösungen und Sirupen eignen sich als Träger z. B. Wasser, Polyole, Saccharose, Invertzucker, Glukose und dergleichen. Für Injektionslösungen eignen sich als Träger z. B. Wasser, Alkohole, Polyole, Glyzerin, pflanzliche Oele etc. Für Suppositorien eignen sich als Träger z. B. natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungs-mittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt, kann man Verbindungen der allgemeinen Formel I und pharmazeutisch annehmbare Säureadditionssalze davon erfindungsgemäss bei der Bekämpfung bzw. Verhütung von Krankheiten verwenden, insbesondere bei der Antagonisierung der zentral dämpfenden, muskelrelaxierenden, ataktischen, blutdrucksenkenden und atemdepressiven Eigenschaften von tranquilisierend wirksamen 1,4-Benzodiazepinen. Insbesondere kann man Verbindungen der allgemeinen Formel I in Kombination mit den weiter oben erwähnten schistosomiazid wirksamen Verbindungen, z. B. in Kombination mit (+)-5-(o-Chlorphenyl)-1,3-dihydro-3-methyl-7-nitro-2H-1,4-benzodiazepin-2-on, bei der Bekämpfung der Schistosomiasis verwenden. Dabei können die Verbindungen der Formel I oder deren pharmazeutisch annehmbare Säureadditionssalze vor, gleichzeitig mit oder nach der Verabreichung bzw. Einnahme von tranquilisierend wirksamen 1,4-Benzodiazepinen verabreicht werden. Wird die Verbindung der Formel I oder ein pharmazeutisch annehmbares Säureadditionssalz davon gleichzeitig mit dem tranquilisierend wirksamen 1,4-Benzodiazepin verabreicht, so kann dies durch Verabreichung als ad-hoc-Kombination oder in Form einer pharmazeutischen Kombination erfolgen, welche eine Verbindung der allgemeinen Formel I oder ein pharmazeutisch annehmbares Säureadditionssalz davon und ein tranquilisierend wirksames 1,4-Benzodiazepin-Derivat enthält ; derartige pharmazeutische Kombinationen sind ebenfalls Gegenstand der vorliegenden Erfindung. Die Dosierung der Verbindungen der Formel I und ihrer pharmazeutisch annehmbaren Säureadditionssalze kann innerhalb weiter Grenzen variieren und ist natürlich in jedem einzelnen Fall den individuellen Gegebenheiten anzupassen. Im allgemeinen dürfte eine Tagesdosis von etwa 0,2 bis etwa 500 mg angemessen sein.

Wie eingangs erwähnt, sind Arzneimittel, enthaltend eine Verbindung der allgemeinen Formel I oder ein pharmazeutisch annehmbares Säureadditionssalz davon, ebenfalls Gegenstand der vorliegenden Erfindung. Solche Arzneimittel, können dadurch hergestellt werden, dass man eine oder mehrere Verbindungen der allgemeinen Formel I oder pharmazeutisch annehmbare Säureadditionssalze davon und gegebenenfalls einen oder mehrere andere therapeutisch wertvolle Stoffe in eine galenische Darreichungsform bringt ; in diesem Zusammenhang sei nochmals auf die weiter oben erwähnten pharmazeutischen Kombinationen hingewiesen, welche ebenfalls Gegenstand der vorliegenden Erfindung sind. Im speziellen sind pharmazeutische Kombinationen, enthaltend eine Verbindung der allgemeinen Formel I und eine der weiter oben erwähnten schistosomiazid wirksamen Verbindungen, insbesondere das (+)-5-(o-Chlorphenyl)-1,3-dihydro-3-methyl-7-nitro-2H-1,4-benzodiazepin-2-on, Gegenstand der vorliegenden Erfindung ; derartige Kombinationen eignen sich zur Bekämpfung von Schistosomiasis.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

Beispiel 1

a) Eine Lösung von 56,5 g 2-Amino-6-nitrobenzoesäure-hydrochlorid in 200 ml entmineralisiertem Wasser und 200 ml Tetrahydrofuran wird unter Rühren auf 10° abgekühlt und während einer Stunde bei 10-30° mit Phosgen behandelt, wobei ein Niederschlag entsteht. Man verdünnt anschliessend mit 300 ml entmineralisiertem Wasser und leitet während etwa 3 Stunden kräftig Luft durch die Suspension (bis die Probe auf Phosgen negativ ist). Der Niederschlag wird abfiltriert, mit Wasser gewaschen und über Phosphorpentoxid im Vakuum getrockent. man erhält 6-Nitroisatosäureanhydrid als beige Kristalle vom Smp. 220-222° (Zersetzung).

b) Ein Gemisch aus 30,0 g 6-Nitroisatosäurenahydrid, 13,5 g Sarcosin und 100 ml Dimethylsufoxid wird unter Rühren während 1,5 Stunden auf 100° erwärmt. Nach Einengen zur Trockene wird der Rückstand in 1,0 l Essigester gelöst. Die organische Phase wird viermal mit je 50 ml gesättigter Natriumbicarbonatlösung und dreimal mit je 100 ml gesättigter Kochsalzlösung gewaschen, wobei die wässrigen Phasen jeweils mit 800 ml Essigester zurückextrahiert werden. Die vereinigten organischen Auszüge werden über Magnesiumsulfat getrocknet und eingedampft. Aus dem Rückstand erhält man nach Umkristallisieren aus Aceton/hexan 3,4-Dihydro-4-methyl-6-nitro-2H-1,4-benzodiazepin-2,5-(1H)-dion, als schwach gelbliche Kristalle vom Smp. 287-289°.

c) Eine Mischung aus 2 g (8,5 mMol) 3,4-Dihydro-4-methyl-6-nitro-2H-1,4-benzodiazepin-2,5-(1H)-dion, 20 ml Chloroform (über basischem Aluminiumoxid filtriert), 10,30 g (85 mMol) Dimethylanilin und 1,94 g (12,7 mMol) Phosphoroxychlorid wird während 2,5 Stunden bei Siedetemperatur gerührt. Die klare Lösung wird auf 6 g Natriumbicarbonat in 60 ml Wasser gegossen und bis zum Ende der Kohlendioxidentwicklung gerührt. Man extrahiert zweimal mit Chloroform. Die organische Phase wird mit Wasser gewaschen, über Magnesiumsulfat getrockent und eingedampft.

Inzwischen kühlt man eine Lösung von 1,44 g (12,8 nMol) Kalium-t-butylat in 10 ml Dimethylformamid auf ca. −40° ab und versetzt sie mit 1,44 g (12,7 mMol) Isocyanessigsäureäthylester. Die erhaltene Lösung versetzt man bei −5° bis 0° tropfenweise mit einer Lösung des obigen Iminchlorids in Dimethylanilin und rührt nach Entfernen des Kühlbades noch während 20 Minuten. Das Reaktionsgemisch wird mit 1,6 ml Essigsäure neutralisiert, auf ca. 200 ml Wasser gegossen und zweimal mit je 50 ml Chloroform ausgeschüttelt. Die organischen Auszüge werden fünfmal mit je 100 ml Wasser gewaschen, über Magnesiumsulfat getrocknet und im Vakuum eingedampft.

Durch Chromatographieren an einer Kieselgelsäule und anschliessendes Umkristalisieren aus Essigester/Hexan erhält man aus dem Rohprodukt reines Aethyl-5,6-dihydro-5-methyl-7-nitro-6-oxo-4H-imidazo [1,5-a] [1,4] benzodiazepin-3-carboxylat vom Smp. 211,5-213°.

Beispiel 2

a) Eine Mischung aus 8,7 g (0,03 Mol) 6-Jodisatosäureanhydrid, 3,2 g (0,036 Mol) Sarcosin und 25 ml Dimethylacetamid wird während 1 Stunde unter Rückfluss zum Sieden erhitzt. Nach Abkühlen und Verdünnen mit Wasser wird das Reaktionsgemisch mit Chloroform ausgeschüttelt. Die Chloroformauszüge werden getrocknet und eingedampft. Nach Umkristallisieren des Rohproduktes aus Methylenchlorid/Aether erhält man 3,4-Dihydro-6-jod-4-methyl-2H-1,4-benzodiazepin-2,5 (1H)-dion vom Schmelzpunkt 214-217°.

b) Eine Mischung aus 5,7 g (0,018 Mol) 3,4-Dihydro-6-jod-4-methyl-2H-1,4-benzodiazepin-2,5 (1H)-dion, 2,4 g (0,026 Mol) Kupfer (I) cyanid und 60 ml Dimethylformamid wird während 45 Minuten auf 50° erhitzt. Nach dem Abkühlen wird das Reaktionsgemisch mit Wasser verdünnt und mehrmals mit Chloroform/Isopropanol (4 : 1) extrahiert. Die vereinigten organischen Auszüge werden über Magnesiumsulfat getrocknet und eingedampft. Das gelbbraune Rohprodukt wird aus Methanol umkristallisiert ; man erhält 6-Cyano-3,4-dihydro-4-methyl-2H-1,4-benzodiazepin-2,5 (1H)-dion vom Schmelzpunkt 253-256° (Zersetzung).

c) Eine Suspension von 0,36 g (9,4 mMol) Natriumhydrid (55-proz. Oeldispersion) in 8 ml trockenem Dimethylformamid wird mit 1,5 g (7,0 mMol) 6-Cyano-3,4-dihydro-4-methyl-2H-1,4-benzodiazepin-2,5(1H)-dion versetzt. Nach beendeter Gasentwicklung kühlt man auf −35° ab, versetzt tropfenweise mit 1,4 ml (8,4 mMol) Diäthylchlorphosphat und rührt das Reaktionsgemisch noch während 15 Minuten bei − 35 bis − 15°.

Inzwischen wird eine Lösung von 0,92 g (8,4 mMol) Kalium-t-butylat in 3 ml trockenem Dimethylformamid in einem Aceton/Trockeneisbad abgekühlt, mit 1,1 ml (8,4 mMol) Isocyanessigsäureäthylester versetzt und dem obigen Reaktionsgemisch zugetropft. Nach Entfernen des Kühlbades wird bei 10° mit 0,48 ml Eisessig neutralisiert, auf 100 ml Wasser gegossen und dreimal mit Chloroform extrahiert. Die organischen Auszüge werden dreimal mit Wasser gewaschen, über Magneisumsulfat getrockent und eingedampft. Das Rohprodukt wird an Kieselgel unter Eluieren mit Chloroform/Methanol (9 : 1) chromatographiert und anschliessend aus Essigester umkristallisiert. Man erhält Aethyl-7-cyano-5,6-dihydro-5-methyl-6-oxo-4H-imidazo [1,5-a] [1,4] benzodiazepin-3-carboxylat vom Schmelzpunkt 200-201°.

Beispiel 3

a) Eine Mischung aus 44,75 g (215 mMol) 5-Nitrosatosäureanhydrid, 21,03 g (236 mMol) Sarcosin und 110 ml Dimethylsulfoxid wird während 1 Stunde bei 100° gerührt. Die braune Lösung wird auf 600 ml Eiswasser gegossen. Man extrahiert mit ca. 300 ml Essigester. Die wässrige Phase wird noch zweimal mit je 100 ml Essigester extrahiert ; die vereinigten organischen Auszüge werden dreimal mit je 80 ml Wasser gewaschen, über Magnesiumsulfat getrocknet und eingedampft. Das verbleibende Oel wird aus ca. 200 ml Aethanol kristallisiert und im Vakuum bei 50° getrockent. Man erhält 3,4-Dihydro-4-methyl-7-nitro-2H-1,4-benzodiazepin-2,5(1H)-dion vom Schmelzpunkt 250-253°. Durch Eindampfen der Mutterlauge und Umkristallisieren des dabei erhaltenen Rückstandes aus Essigester erhält man noch eine weitere Portion des obigen Materials vom Schmelzpunkt 250-252°.

b) Eine unter Argon gerührte Suspension von 16,0 g (68 mMol) 3,4-Dihydro-4-methyl-7-nitro-2H-1,4-benzodiazepin-2,5(1H)-dion in 100 ml Dimethylformamid wird mit 9,12 g (81,6 mMol) Kalium-t-butylat versetzt, auf − 30° abgekühlt und bei dieser Temperatur tropfenweise mit 10,2 ml (71,4 mMol) Diäthylchlorphosphat versetzt. Man rührt noch während 0,25 Stunden bei − 30°.

Inzwischen wird eine Lösung von 8,38 g (74,8 mMol) Kalium-t-butylat in 25 ml Dimethylformamid in einem Aceton/Trockeneisbad abgekühlt,mit 8,5 ml (74,8 mMol) Isocyan-essigsäure-äthylester versetzt und bei − 30 bis − 15° dem obigen Reaktionsgemisch zugetropft. Man entfernt das Kühlbad, rührt bis die Temperatur 25° erreicht hat,neutralisiert mit ca. 2 ml Eisessig,giesst auf ca. 400 ml Wasser und extrahiert dreimal mit je ca. 150 ml Chloroform. Die vereinigten Chloroformauszüge werden zweimal mit je ca. 80 ml Wasser gewaschen, über Magnesiumsulfat getrocknet und im Vakuum eingedampft.

Reinigung des Rohproduktes durch Chromatographieren an einer Kieselgelsäule und anschliessendes Umkristallisieren aus Essigester liefert Aethyl-5,6-dihydro-5-methyl-8-nitro-6-oxo-4H-imidazo [1,5-a] [1,4] benzodiazepin-3-carboxylat vom Schmelzpunkt 197-199°.

Beispiel 4

a) 300 ml 89-proz. Salpetersäure werden bei Raumtemperatur über einen Zeitraum von 35 Minuten portionenweise mit 94,5 g (496,8 mMol) 3,4-Dihydro-4-methyl-2H-1,4-benzodiazepin-2,5(1H)-dion versetzt. Das Reaktionsgemisch wird 18 Stunden bei Raumtemperatur gerührt und auf 3,5 l Eiswasser gegossen. Das ausgefallene Material wird abgenutscht, mit Wasser gewaschen und im Vakuum bei 70° getrocknet. Man erhält 3,4-Dihydro-4-methyl-7-nitro-2H-1,4-benzodiazepin-2,5(1H)-dion vom Schmelzpunkt 252-253°.

b) In Analogie zu den Angaben in Beispiel 3b) erhält man aus obigem Material Aethyl-5,6-dihydro-5-methyl-8-nitro-6-oxo-4H-imidazo [1,5-a] [1,4] benzodiazepin-3-carboxylat vom Schmelzpunkt 197-199°.

Beispiel 5

a) Unter einer Argonatmosphäre werden 19,0 g (0,10 Mol) 3,4-Dihydro-4-methyl-2H-1,4-benzodiazepin-2,5 (1H)-dion in 100 ml trockenem Dimethylformamid vorgelegt. Man gibt 15,5 g (0,12 Mol) Kalium-t-butylat zu, wobei die Temperatur von 25° auf 39° steigt. Man kühlt auf Raumtemperatur und tropft zwischen 18-22° 18,2 g (0,105 Mol) Diäthylchlorphosphat zu.

Separat werden 11,2 g (0,10 Mol) Kalium-t-butylat in 30 ml Dimethylformamid gelöst. Diese Lösung wird auf ca. − 50° gekühlt und unter Argon mit 11,3 g (0,10 Mol) Isocyanessigsäureäthylester versetzt. Anschliessend wird diese Lösung bei 18 bis 23° unter Kühlung zum obigen Reaktionsgemisch getropft. Man rührt während 1 Stunde bei Raumtemperatur, gibt 5 ml Essigsäure zu, giesst dann auf 500 ml Wasser und extrahiert zweimal mit je 200 ml Chloroform. Die vereinigten Chloroformauszüge werden dreimal mit je 300 ml Wasser gewaschen, über Magnesiumsulfat getrocknet und eingedampft. Zum öligen Rückstand gibt man 150 ml Essigester und lässt bei 0° kreistallisieren. Man nutscht die ausgeschiedenen Kristalle ab, wäscht sie mit kaltem Essigester und erhält Aethyl-5,6-dihydro-5-methyl-6-oxo-4H-imidazo [1,5-a] [1,4] benzodiazepin-3-carboxylat vom Schmelzpunkt 163-165°. Nach Umkristallisieren aus 50 ml Essigester zeigt das Produkt einen Schmelzpunkt von 164-165°.

b) 2,85 g Aethyl-5,6-dihydro-5-methyl-6-oxo-4H-imidazo [1,5-a] [1,4] benzodiazepin-3-carboxylat werden in einer Mischung aus 10 ml 98-proz. Salpetersäure und 15 ml 98-proz. Schwefelsäure gelöst und während 2 Stunden auf 120° erhitzt. Anschliessend kühlt man die erhaltene Mischung auf Raumtemperatur ab, giesst auf Eis, neutralisiert mit ca. 25-proz. Ammoniak und extrahiet mit Chloroform. Die Chloroformauszüge werden über Magnesiumsulfat getrocknet und eingedampft. Nach Umkristallisieren des Rückstandes aus Essigester erhält man Aethyl-5,6-dihydro-5-methyl-8-nitro-6-oxo-4H-imidazo [1,5-a] [1,4] benzodiazepin-3-carboxylat vom Schmelzpunkt 197-198°.

Beispiel 6

a) 130 ml 89-proz. Salpetersäure werden bei Raumtemperatur über einen Zeitraum von 1,5 Stunden portionenweise mit 43,0 (199 mMol) (S)-1,2,3,11a-Tetrahydro-5H-pyrrolo [2,1-c] [1,4] benzodiazepin-5,11(10H)-dion versetzt. Das Reaktionsgemisch wird 18 Stunden bei Raumtemperatur gerührt und auf 1,5 l Eiswasser gegossen. Das ausgefallene Material wird abgenutscht, mit viel Wasser gewaschen und im

Vakuum bei 80° getrocknet. Man eerhält (S)-(+)-1,2,3,11a-Tetra-hydro-7-nitro-5H-pyrrolo [2,1-c] [1,4] benzodiazepin-5,11(10H)-dion vom Schmelzpunkt 259-261°.

b) Eine auf 5° abgekühlte Lösung 22,0 g (84,2 mMol) (S)-(+)-1,2,3,11a-Tetrahydro-7-nitro-5H-pyrrolo [2,1-c] [1,4] benzodiazepin-5,11(10H)-dion in 100 ml Dimethylformamid wird mit 11,3 g (101 mMol) Kalium-t-butylat versetzt. Zu der erhaltenen dunkelroten Lösung werden bei – 40° 12,6 ml (88,4 mMol) Diäthylchlorphosphat getropft. Man rührt noch ca. 20 Minuten bei – 20 bis – 10°.

Inzwischen wird eine Lösung von 10,4 g (92,6 mMol) Kalium-t-butylat in 30 ml Dimethylformamid einem Aceton/Trockeneisbad abgekühlt, mit 10,5 ml (92,6 mMol) Isocyanessigsäureäthylester versetzt und bei – 20 bis – 10° dem obigen Reaktionsgemisch zugetropft. Man entfernt das Kühlbad, rührt, bis die Temperatur 20° erreicht hat, neutralisiert mit 3 ml Eisessig, giesst auf ca. 300 ml Eiswasser und extrahiert viermal mit je ca. 150 ml Chloroform. Die vereinigten Chloroformauszüge werden dreimal mit je ca. 50 ml gesättigter Kochsalzlösung gewaschen, über Magnesiumsulfat getrocknet und eingedampft.

Nach Reinigung des Rohproduktes durch Chromatographieren an einer Kieselgelsäule und anschliessendes Umkristallisieren aus Essigester erhält man Aethyl-(S)-(+)-11,12,13,-13a-tetrahydro-7-nitro-9-oxo-9H-imidazo [1,5-a] pyrrolo-[2,1-c] [1,4] benzodiazepin-1-carboxylat vom Schmelzpunkt 191-193°.

Beispiel 7

a) Unter einer Argonatmosphäre versetzt man eine Lösung von 21,6 g (0,10 Mol) (S)-(+)-1,2,3-11a-Tetrahydro-5H-pyrrolo [2,1-c] [1,4] benzodiazpin-5,11(10H)-dion in 100 ml trockenem Dimethylformamid mit 13,5 g (0,12 Mol) Kalium-t-butylat, wobei die Temperatur von 24° auf 46° steigt. Man kühlt auf Raumtemperatur und tropft zwischen 18 bis 23° 18,2 g (0,105 Mol) Diäthylchlorphosphat zu.

Separat werden 11,2 g (0,10 Mol) Kalium-t-butylat in 30 ml Dimethylformamid gelöst. Diese Lösung wird auf ca. –50° gekühlt und unter Argon mit 11,3 g (0,10 Mol) Isocyanessigsäureäthylester versetzt. Anschliessend wird diese Lösung bei 18 bis 23° unter Kühlung zum obigen Reaktionsgemisch getropft. Man rührt während 1 Stunde bei Raum-temperatur, gibt 5 ml Essigsäure zu, giesst dann auf 500 ml Wasser und extrahiert zweimal mit je 200 ml Chloroform. Die vereinigten Chloroformphasen werden dreimal mit je 300 ml Wasser gewaschen, über Magnesiumsulfat getrocknet und eingedampft. Zum öligen Rückstand gibt man 150 ml Essigsäure und lässt bei 0° kristallisieren. Man nutscht die ausgeschiedenen Kristalle ab, wäscht mit kaltem Essigester und erhält Aethyl-(S)-11,12,13,13a-tetrahydro-9-oxo-9H-imidazo [1,5-a] pyrrolo [2,1-c] [1,4] benzodiazepin-1-carboxylat vom Schmelzpunkt 196-197°. Die Mutterlauge wird eingedampft und der Rückstand wird in 50 ml Essigester gelöst. Daraus kristallisiert eine weitere Portion des obigen Produkts ; Schmelzpunkt 195-196°.

b) Eine Lösung von 3,11 g Aethyl-(S)-11,12,13,13a-tetrahydro-9-oxo-9H-imidazo [1,5-a] pyrrolo [2,1-c] [1,4] benzodiazepin-1-carboxylat in einer Mischung aus 10 ml 98-proz. Salpetersäure und 15 ml 98-proz. Schwefelsäure wird während 6 Stunden auf 100° erhitzt. Anschliessend kühlt man auf Raumtemperatur ab, giesst auf Eis, neutralisiert mit ca. 25-proz. Ammoniak und extrahiert mit Chloroform. Die Chloroformauszüge werden über Magnesiumsulfat getrocknet und eingedampft. Der Rückstand wird aus Essigester umkristallisiert und liefert Aethyl-(S)-11,12,13,13a-tetrahydro-7-nitro-9-oxo-9H-imidazo [1,5-a] pyrrolo [2,1-c] [1,4] benzodiazepin-1-carboxylat vom Schmelzpunkt 193-194°.

Beispiel 8

a) Eine Mischung aus 39,5 g 5-Brom-isatosäureanhydrid, 14,5 g Sarcosin und 150 ml Dimethylsulfoxid wird unter Rühren auf 100° erwärmt. Man rührt nach beendeter Gasentwicklung noch 30 Minuten bei 100° und giesst anschliessend auf 900 ml Eiswasser. Die ausgefallenen Kristalle werden abgenutscht, mit Wasser gewaschen und im Vakuum bei 50° über Phosphorpentoxid getrocknet. Man erhält 7-Brom-3,4-dihydro-4-methyl-2H-1,4-benzodiazepin-2,5(1H)-dion, als hellbeige Kristalle. Eine aus Methanol umkristallisierte Probe hat einen Schmelzpunkt von 260-261°.

b) 15,0 g 7-Brom-3,4-dihydro-4-methyl-2H-1,4-benzodiazepin-2,5(1H)-dion und 7,5 g Kupfer (I) cyanid (frisch hergestellt) werden in einer Reibschale gut vermischt und mit 7,2 ml trockenem Pyridin (frisch über Kaliumhydroxid destilliert) unter Argon über einen Zeitraum von 3 Stunden von 165° auf 195° erhitzt. Nach Abkühlen auf 150° wird die feste Masse in ca. 250 ml siedendem Dimethylformamid gelöst. Man lässt abkühlen, giesst auf 800 ml Eiswasser und extrahiert dreimal mit je 500 ml Chloroform. Die organischen Auszüge werden mit 250 ml 2N Salzsäure und dreimal mit je 500 ml Wasser gewaschen, in Gegenwart von Aktivkohle getrocknet und eingedampft. Der Rückstand wird in ca. 1 l Methanol gelöst. Die Lösung wird auf ein Volumen von 250 ml eingeengt, mit Aktivkohle behandelt und auf 5° abgekühlt. Das ausgefallene Material wird abgenutscht und getrocknet ; man erhält 7-Cyano-3,4-dihydro-4-methyl-2H-1,4-benzodiazepin-2,5(1H)-dion als weisse Kristalle vom Schmelzpunkt 256-258°.

c) Eine Lösung von 5 g (23 mMol) 7-Cyano-3,4-dihydro-4-methyl-2H-1,4-benzodiazepin-2,5(1H)-dion in 25 ml Dimethylformamid versetzt man unter eine Argonatmosphäre mit 0,96 g (25 mMol) Natriumhydrid (60-proz. Oeldispersion) und rührt während 1 Stunde. Der erhaltenen Suspension tropft man bei –20° 4,31 g (25 mMol) Diäthylchlorphosphat zu und rührt 10 Minuten bei dieser Temperatur.

Inzwischen kühlt man eine Lösung von 2,80 g (25 mMol) Kalium-t-butylat in 10 ml Dimethylformamid in einem Aceton/Trockeneisbad ab und versetzt sie mit 2,83 g (25 Mol) Isocyanessigsäureäthylester. Diese

Lösung wird bei – 10 bis – 20° dem obigen Reaktionsgemisch zugetropft. Man rührt noch 0,5 Stunden ohne Kühlung, neutralisiert mit 2,5 ml Essigsäure, giesst auf ca. 250 ml Wasser und extrahiert dreimal mit Chloroform. Die organischen Auszüge werden fünfmal mit Wasser gewaschen, über Magnesiumsulfat getrocknet und eingedampft. Nach Reinigung des Rohproduktes durch Säulenchromatographie an Kieselgel (Elutionsmittel Essigester) und anschliessendes Umkristallisieren aus Essigester erhält man Aethyl-8-cyano-5,6-dihydro-5-methyl-6-oxo-4H-imidazo [1,5-a] [1,4] benzodiazepin-3-carboxylat vom Schmelzpunkt 183,5-184,5°.

Aethyl-5,6-dihydro-5-methyl-7-nitro-6-oxo-4H-imidazo [1,5-a] [1,4] benzodiazepin-3-carboxylat (Wirkstoff A) kann wie in den Beispielen A bis G angegeben als Wirkstoff zur Herstellung von pharmazeutischen Präparaten verwendet werden :

Beispiel A

Es werden in üblicher Weise Tabletten folgender Zusammensetzung hergestellt :

| | pro Tablette |
|---|---|
| Wirkstoff A | 1 mg |
| Milchzucker | 103 mg |
| Maisstärke | 25 mg |
| Mikrokristalline Cellulose | 70 mg |
| Magnesiumstearat | 1 mg |
| Total | 200 mg |

Beispiel B

Es werden Kapseln folgender Zusammensetzung hergestellt :

| | pro Kapsel |
|---|---|
| Wirkstoff A | 1 mg |
| Milchzucker | 164 mg |
| Maisstärke | 30 mg |
| Talk | 5 mg |
| Total | 200 mg |

Der Wirkstoff, Milchzucker und Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapseln abgefüllt.

Beispiel C

Es werden Injektionslösungen folgender Zusammensetzung hergestellt :

| | pro ml | |
|---|---|---|
| Wirkstoff A | 0,5 | mg |
| Benzylalkohol | 0,015 | ml |
| Propylenglykol | 0,4 | ml |
| Aethanol (95-proz.) | 0,1 | ml |
| Natriumbenzoat | 48,8 | mg |
| Benzoesäure | 1,2 | mg |
| Wasser für Injektion q.s. ad | 1,0 | ml |

Zur Herstellung von 10 000 ml Injektionslösung löst man 5 g des Wirkstoffes in 150 ml Benzylalkohol und gibt 4 000 ml Propylenglykol und 1 000 ml Aethanol zu. Dann löst man 12 g Benzoesäure in dem obigen Gemisch und gibt eine Lösung von 488 g Natriumbenzoat in 300 ml Wasser (für Injektion) zu. Die erhaltene Lösung wird durch Zugabe von Wasser (für Injektion) auf ein Volumen von 10 000 ml gebracht, filtriert und in Ampullen geeigneter Grösse abgefüllt ; man füllt das Restvolumen der Ampullen mit Stickstoff, schmilzt die Ampullen zu und sterilisiert sie während 30 Minuten im Autoklaven bei 0,7 Atm.

Beispiel D

Es werden Suppositorien folgender Zusammensetzung hergestellt :

| | pro Supp. |
|---|---|
| Wirkstoff A | 0,001 g |
| Cacaobutter (Smp. 36-37°) | 1,255 g |
| Carnauba-Wachs | 0,044 g |
| Total | 1,3 g |

Cacaobutter und Carnauba-Wachs werden in einem Glasoder Stahlgefäss geschmolzen, gründlich vermengt und auf 45° abgekühlt. Hierauf gibt man den fein pulverisierten Wirkstoff zu und rührt, bis es ɔllständig dispergiert ist. Man giesst die Mischung in Suppositorienformen geeigneter Grösse, lässt abkühlen, nimmt dann die Suppositorien aus den Formen und verpackt sie inzeln in Wachspapier oder Metallfolien.

Beispiel E

Es werden Kapseln folgender Zusammensetzung hergestellt :

| | mg/Kapsel |
|---|---|
| Wirkstoff A | 20,0 |
| (+)-5-(o-Chlorphenyl)-1,3-dihydro-3-methyl 7-nitro-2H-1,4-benzodiazepin-2-on (Wirkstoff B) | 30,0 |
| Milchzucker krist. | 100,0 |
| Maisstärke weiss | 27,5 |
| Talk | 10,0 |
| Magnesiumstearat | 2,5 |
| Total | 190,0 |

Die beiden Wirkstoffe werden mit den Hilfsstofen gut vermischt und zu je 190,0 mg in Steckkapseln geeigneter Grösse abgefüllt.

Beispiel F

Es werden Tabletten folgender Zusammensetzung hergestellt :

| | mg/Tablette |
|---|---|
| Wirkstoff A | 10,0 |
| (+)-5-(o-Chlorphenyl)-1,3-dihydro-3-methyl-7-nitro-2H-1,4-benzodiazepin-2-on (Wirkstoff B) | 30,0 |
| Milchzucker pulvis | 15,0 |
| Maisstärke weiss | 19,5 |
| Povidon K30 | 3,5 |
| Maisstärke weiss | 10,0 |
| Magnesiumstearat | 2,0 |
| Total | 90,0 |

Die beiden Wirkstoffe, Milchzucker pulvis und die erste Portion Maisstärke weiss werden gemischt und gesiebt. Diese Mischung wird mit einer Lösung von Povidon K30 in Wasser befeuchtet, geknetet, granuliert, getrocknet und gesiebt. Dem Granulat werdendie zweite Portion Maisstärke weiss und Magnesiumstearat zugesetzt. Nach Mischen wird die erhaltene Masse zu 90 mg schweren Tabletten verpresst.

Beispiel G

Es werden Tabletten folgender Zusammensetzung hergestellt :

| | mg/Tablette |
|---|---|
| Wirkstof A | 30 |
| (+)-5-(o-Chlorphenyl)-1,3-dihydro-3-methyl-7-nitro-2H-1,4-benzodiazepin-2-on (Wirkstoff B) | 30 |
| Milchzucker pulvis | 22 |
| Maisstärke weiss | 22 |
| Povidon K30 | 6 |
| Maisstärke weiss | 16 |
| Magnesiumstearat | 4 |
| Total | 130 |

Die beiden Wirkstoffe, Milchzucker pulvis und die erste Portion Maisstärke weiss werden gemischt und gesiebt. Diese Mischung wird mit einer Lösung von Povidon K30 in Wasser befeuchtet, geknetet, granuliert, getrocknet und gesiebt. Dem Granulat werden die zweite Portion Maisstärke weiss und Magneisumstearat zugesetzt. Nach Mischen wird die erhalten Masse zu 130 mg schweren Tabletten verpresst.

**Patentansprüche**

1. Imidazodiazepine der allgemeinen Formel

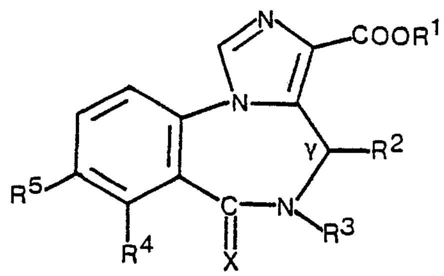

(I)

worin $R^1$ Methyl, Aethyl oder Isopropyl, einer der Reste $R^4$ und $R^5$ Wasserstoff und der andere Nitro oder Cyano bedeuten, und entweder $R^2$ Wassestoff und $R^3$ Wasserstoff oder $C_1$-$C_7$ Alkyl bedeuten, oder $R^2$ und $R^3$ zusammen Dimethylen, Trimethylen oder Propenylen bedeuten und das mit $\gamma$ bezeichnete Kohlenstoffatom die (S)- oder (R,S)-Konfiguration aufweist, und X ein Sauerstoff- oder Schwefelatom bedeutet, und pharmazeutisch annehmbare Säureadditionssalze davon.

2. Verbindungen gemäss Anspruch 1, dadurch gekennzeichnet, dass $R^3$ Methyl bedeutet, wenn $R^2$ Wasserstoff bedeutet.

3. Verbindungen gemäss Anspruch 1, dadurch gekennzeichnet, dass das in Formel I mit $\gamma$ bezeichnete Kohlenstoffatom die (S)-Konfiguration aufweist, wen $R^2$ und $R^3$ zusammen Dimethylen oder Trimethylen bedeuten.

4. Aethyl-5,6-dihydro-5-methyl-7-nitro-6-oxo-4H-imidazo [1,5-a] [1,4] benzodiazepin-3-carboxylat.

5. Aethyl-7-cyano-5,6-dihydro-5-methyl-6-oxo-4H-imidazo [1,5-a] [1,4] benzodiazepin-3-carboxylat.

6. Aethyl-5,6-dihydro-5-methyl-8-nitro-6-oxo-4H-imidazo [1,5-a] [1,4] benzodiazepin-3-carboxylat, Aethyl-8-cyano-5,6-dihydro-5-methyl-6-oxo-4H-imidazo [1,5-a] [1,4]-benzodiazepin-3-carboxylat und Aethyl-(S)-(+)-11,12,13,13a-tetrahydro-7-nitro-9-oxo-9H-imidazo [1,5-a] pyrrolo [2,1-c]-[1,4] benzodiazepin-1-carboxylat.

7. Verbindungen der allgemeinen Formel

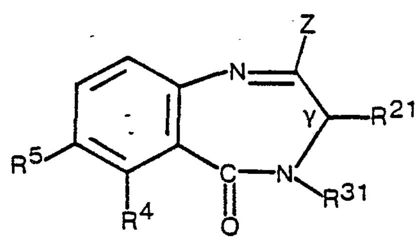

(II)

worin entweder $R^{21}$ Wasserstoff und $R^{31}$ $C_1$-$C_7$ Alkyl bedeuten, oder $R^{21}$ und $R^{31}$ zusammen Dimethylen, Trimethylen oder Propenylen bedeuten und das mit $\gamma$ bezeichnete Kohlenstoffatom die (S)- oder (R,S)-Konfiguration aufweist, einer der Reste $R^4$ und $R^5$ Wasserstoff und der andere Nitro oder Cyano und Z eine Abgangsgruppe bedeuten, mit der Massgabe, dass $R^4$ bzw. $R^5$ nicht Nitro bedeutet, wenn $R^{21}$ Wasserstoff und $R^{31}$ $C_1$-$C_7$ Alkyl bedeuten.

8. Verbindungen der allgemeinen Formel

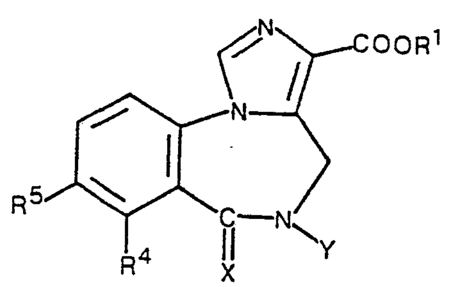

(V)

worin $R^1$ Methyl, Aethyl oder Isopropyl, einer der Reste $R^4$ und $R^5$ Wasserstoff und der andere Nitro oder Cyano, X ein Sauerstoff- oder Schwefelatom und Y eine Schutzgruppe bedeuten.

9. Verbindungen der allgemeinen Formel

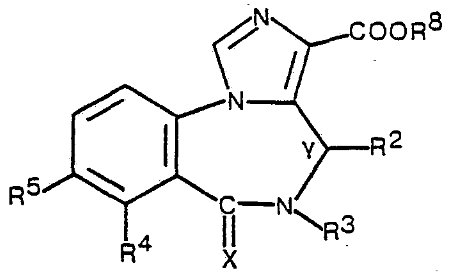

(VII)

worin einer der Reste $R^4$ und $R^5$ Wasserstoff und der andere Nitro oder Cyano bedeutet, und entweder $R^2$ Wasserstoff und $R^3$ Wasserstoff oder $C_1$-$C_7$ Alkyl bedeuten, oder $R^2$ und $R^3$ zusammen Dimethylen, Trimethylen oder Propenylen bedeuten und das mit γ bezeichnete Kohlenstoffatom die (S)- oder (R,S)-Konfiguration aufweist, X ein Sauerstoff oder Schwefelatom und $R^8$ $C_1$-$C_7$ Alkyl, jedoch nicht Methyl, Aethyl oder Isopropyl, bedeuten.

10. Verbindungen gemäss einem der Ansprüche 1 bis 6 als pharmazeutische Wirkstoffe.

11. Verbindungen gemäss einem der Ansprüche 1 bis 6 als Wirkstoffe, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

12. Verfahren zur Herstellung von Verbindungen gemäss einem der Ansprüche 1 bis 6, dadurch gekennzeichnet, dass man

a) eine Verbindung der allgemeinen Formel

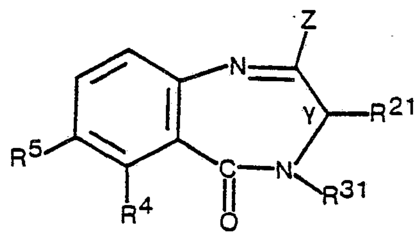

(II)

worin $R^4$ und $R^5$ die in Anspruch 1 angegebene Bedeutung besitzen und Z eine Abgangsgruppe bedeutet, und entweder $R^{21}$ Wasserstoff und $R^{31}$ $C_1$-$C_7$ Alkyl bedeuten, oder $R^{21}$ und $R^{31}$ zusammen Dimethylen, Trimethylen oder Propenylen bedeuten und das mit γ bezeichnete Kohlenstoffatom die (S)- oder (R,S)-Konfiguration aufweist, in Gegenwart einer Base mit einem Isocyanessigester der allgemeinen Formel

$$CN—CH_2—COOR^1 \qquad (III)$$

worin $R^1$ die in Anspruch 1 angegebene Bedeutung besitzt, umsetzt, oder

b) in einer Verbindung der allgemeinen Formel

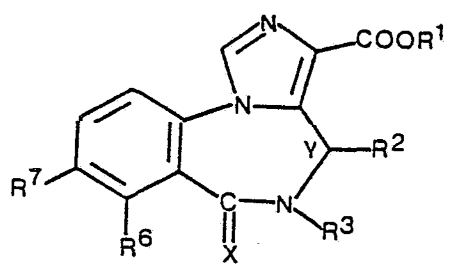

(IV)

worin einer der Reste $R^6$ und $R^7$ Wasserstoff und der andere Halogen bedeutet, und $R^1$, $R^2$, $R^3$ und X die in Anspruch 1 angegebene Bedeutung besitzen, das Halogenatom durch die Cyanogruppe ersetzt, oder

c) eine Verbindung der allgemeinen Formel

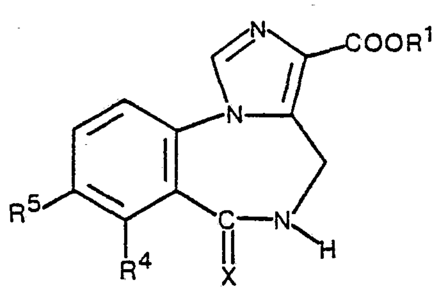

(Ia)

worin $R^1$, $R^4$, $R^5$ und X die in Anspruch 1 angegebene Bedeutung besitzen, an der sekundären Aminogruppe entsprechend substituiert, oder

d) in einer Verbindung der allgemeine Formel

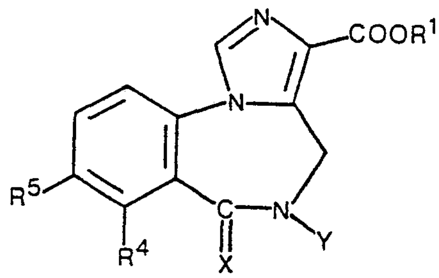

(V)

worin $R^1$, $R^4$, $R^5$ und X die in Anspruch 1 angegebene Bedeutung besitzen, und Y eine Schutzgruppe bedeutet, die Schutzgruppe abspaltet, oder

e) eine Verbindung der allgemeinen Formel

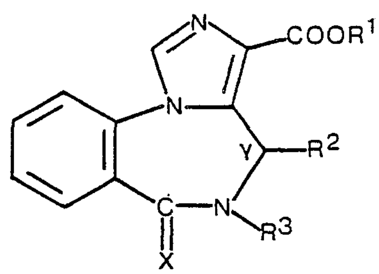

(VI)

worin $R^1$, $R^2$, $R^3$ und X die in Anspruch 1 angegebene Bedeutung besitzen, nitriert, oder

f) eine Verbindung der allgemeinen Formel

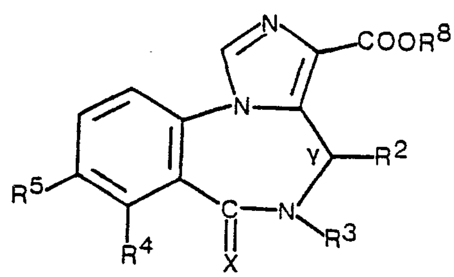

(VII)

worin $R^2$, $R^3$, $R^4$, $R^5$ und X die in Anspruch 1 angegebene Bedeutung besitzen, und $R^8$ $C_1$-$C_7$ Alkyl bedeutet, umestert, oder

g) in einer Verbindung der allgemeinen Formel

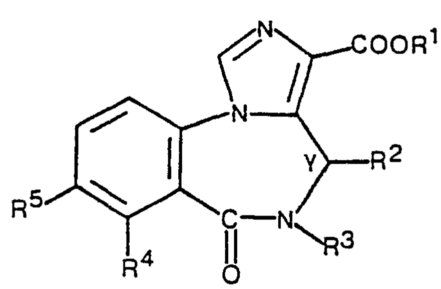

(Ib)

worin $R^1$, $R^2$, $R^3$, $R^4$ und $R^5$ die in Anspruch 1 angegebene Bedeutung besitzen, die Carbonyl-Gruppe in die Thiocarbonyl-Gruppe überführt, und

h) erwünschtenfalls eine erhaltene Verbindung der allgemeinen Formel I in ein pharmazeutisch annehmbares Säureadditionssalz überführt.

13. Arzneimittel, enthaltend eine Verbindung gemäss einem der Ansprüche 1 bis 6.

14. Arzneimittel gemäss Anspruch 13, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

15. Schistosomizid wirksames Arzneimittel, enthaltend eine Verbindung gemäss einem der Ansprüche 1 bis 6 und (+)-5-(o-Chlorphenyl)1,3-dihydro-3-methyl-7-nitro-2H-1,4-benzodiazepin-2-on.

## Claims

1. Imidazodiazepines of the general formula

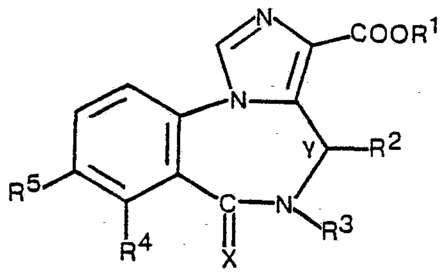

(I)

wherein $R^1$ signifies methyl, ethyl or isopropyl, one of the residues $R^4$ and $R^5$ signifies hydrogen and the other signifies nitro or cyano, and either $R^2$ signifies hydrogen and $R^3$ signifies hydrogen or $C_1$-$C_7$ alkyl, or $R^2$ and $R^3$ together signify dimethylene, trimethylene or propenylene and the carbon atom denoted by $\gamma$ has the (S)- or (R,S)-configuration, and X signifies an oxygen or sulphur atom, and pharmaceutically acceptable acid addition salts thereof.

2. Compounds in accordance with claim 1, characterized in that $R^3$ signifies methyl when $R^2$ signifies hydrogen.

3. Compounds in accordance with claim 1, characterized in that the carbon atom denoted by $\gamma$ in formula I has the (S)-configuration when $R^2$ and $R^3$ together signify dimethylene or trimethylene.

4. Ethyl 5,6-dihydro-5-methyl-7-nitro-6-oxo-4H-imidazo [1,5-a] [1,4] benzodiazepine-3-carboxylate.

5. Ethyl 7-cyano-5,6-dihydro-5-methyl-6-oxo-4H-imidazo [1,5-a] [1,4] benzodiazepine-3-carboxylate.

6. Ethyl 5,6-dihydro-5-methyl-8-nitro-6-oxo-4H-imidazo [1,5-a] [1,4] benzodiazepine-3-carboxylate, ethyl 8-cyano-5,6-dihydro-5-methyl-6-oxo-4H-imidazo [1,5-a] [1,4] benzodiazepine-3-carboxylate and ethyl (S)-(+)-11,12,13,13a-tetrahydro-7-nitro-9-oxo-9H-imidazo [1,5-a] pyrrolo [2,1-c] [1,4]-benzodiazepine-1-carboxylate.

7. Compounds of the general formula

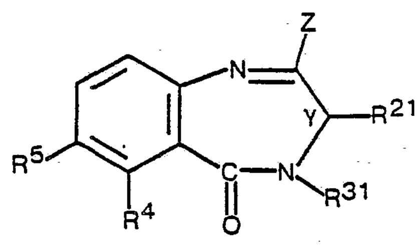

(II)

wherein either $R^{21}$ signifies hydrogen and $R^{31}$ signifies $C_1$-$C_7$ alkyl, or $R^{21}$ and $R^{31}$ together signify dimethylene, trimethylene or propenylene and the carbon atom denoted by $\gamma$ has the (S)- or (R,S)-configuration, one of the residues $R^4$ and $R^5$ signifies hydrogen and the other signifies nitro or cyano and Z signifies a leving group, with the proviso that $R^4$ or $R^5$ does not signify hydrogen when $R^{21}$ signifies hydrogen and $R^{31}$ signifies $C_1$-$C_7$ alkyl.

8. Compounds of the general formula

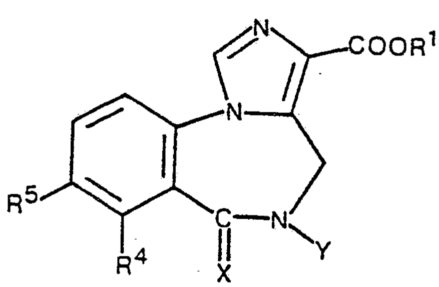

(V)

wherein $R^1$ signifies methyl, ethyl or isopropyl, one of the residues $R^4$ and $R^5$ signifies hydrogen and the other signifies nitro or cyano, X signifies an oxygen or sulphur atom and Y signifies a protecting group.

9. Compounds of the general formula

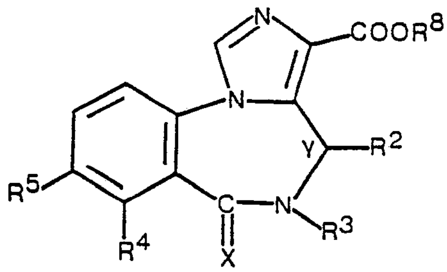

(VII)

wherein one of the residues $R^4$ and $R^5$ signifies hydrogen and the other signifies nitro or cyano, and either $R^2$ signifies hydrogen and $R^3$ signifies hydrogen or $C_1$-$C_7$ alkyl, or $R^2$ and $R^3$ together signify dimethylene, trimethylene or propenylene and the carbon atom denoted by $\gamma$ has the (S)- or (R,S)-configuration, X signifies an oxygen or sulphur atom and $R^8$ signifies $C_1$-$C_7$ alkyl, but does not signify methyl, ethyl or isopropyl.

10. Compounds in accordance with any one of claims 1 to 6 as pharmaceutically active substances.

11. Compounds in accordance with any one of claims 1 to 6 as active substances which antagonize the central-depressant, muscle relaxant, ataxic, blood pressure-lowering and respiratory-depressant properties of 1,4-benzodiazepines which have tranquillizing activity.

12. A process for the manufacture of compounds in accordance with any one of claims 1 to 6, characterized by

a) reacting a compound of the general formula

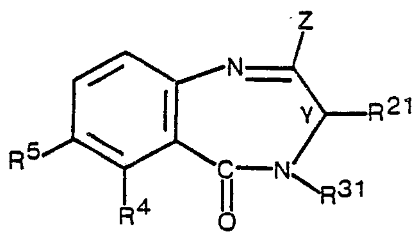

(II)

wherein $R^4$ and $R^5$ have the significance given in claim 1 and Z signifies a leaving group, and either $R^{21}$ signifies hydrogen and $R^{31}$ signifies $C_1$-$C_7$ alkyl, or $R^{21}$ and $R^{31}$ together signify dimethylene, trimethylene or propenylene and the carbon atom denoted by $\gamma$ has the (S)- or (R,S)-configuration, in the presence of a base with an isocyanoacetic ester of the general formula

$$CN—CH_2—COOR^1 \qquad (III)$$

wherein $R^1$ has the significance given in claim 1, or

b) replacing the halogen atom in a compound of the general formula

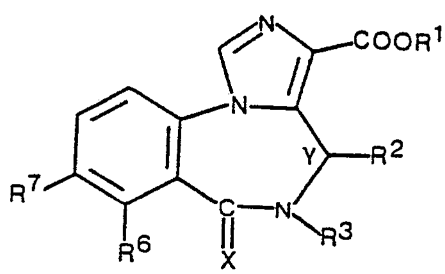

(IV)

wherein one of the residues $R^6$ and $R^7$ signifies hydrogen and the other signifies halogen, and $R^1$, $R^2$, $R^3$ and X have the significance given in claim 1, by the cyano group, or

c) appropriately substituting a compound of the general formula

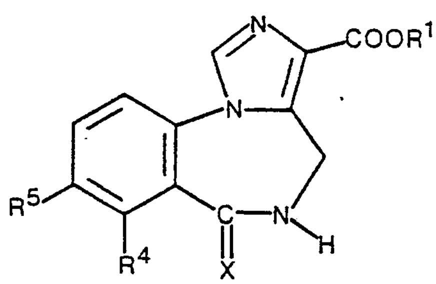

(Ia)

wherein $R^1$, $R^4$, $R^5$ and X have the significance given in claim 1, at the secondary amino group, or

d) cleaving off the protecting group in a compound of the general formula

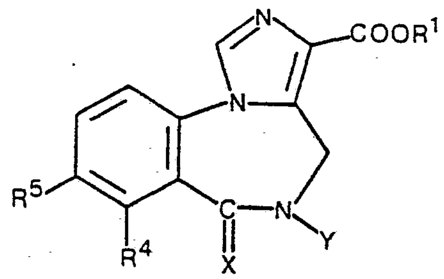

(V)

wherein $R^1$, $R^4$, $R^5$ and X have the significance given in claim 1, and Y signifies a protecting group, or

e) nitrating a compound of the general formula

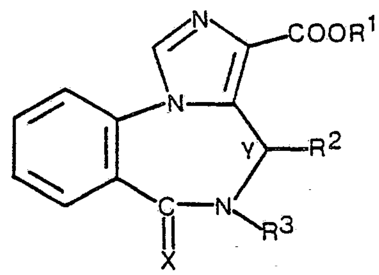

(VI)

wherein $R^1$, $R^2$, $R^3$ and X have the significance given in claim 1, or

f) trans-esterifying a compound of the general formula

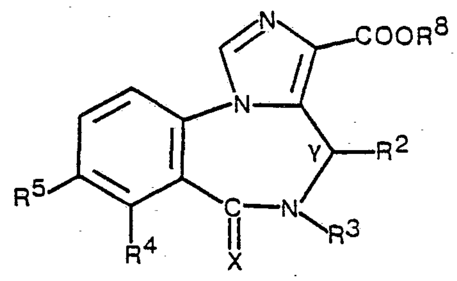

(VII)

wherein $R^2$, $R^3$, $R^4$, $R^5$ and X have the significance given in claim 1, and $R^8$ signifies $C_1$-$C_7$ alkyl, or

g) converting the carbonyl group in a compound of the general formula

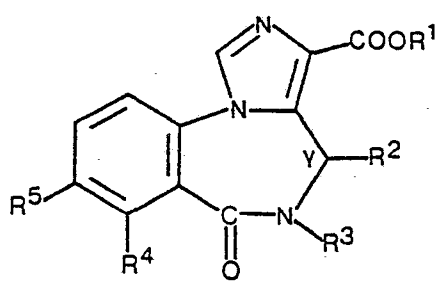

(Ib)

wherein $R^1$, $R^2$, $R^3$, $R^4$ and $R^5$ have the significance given in claim 1, into the thiocarbonyl group, and

h) if desired, converting a compound of general formula I obtained into a pharmaceutically acceptable acid addition salt.

13. A medicament containing a compound in accordance with any one of claims 1 to 6.

14. A medicament in accordance with claim 13, which antagonizes the central-depressant, muscle relaxant, ataxic, blood pressure-lowering and respiratory-depressant properties of 1,4-benzodiazepines which have tranquillizing activity.

15. A schistosomicidally-active medicament containing a compound in accordance with any one of claims 1 to 6 and (+)-5-(o-chlorophenyl)-1,3-dihydro-3-methyl-7-nitro-2H-1,4-benzodiazepin-2-one.

**Revendications**

1. Imidazodiazépines de formule générale

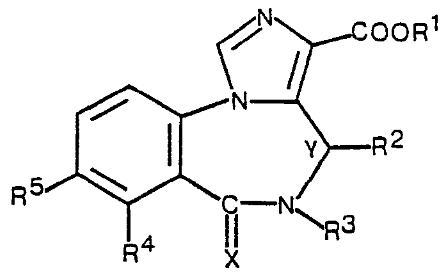

(I)

où $R^1$ représente un méthyle, un éthyle ou un isopropyle, l'un des radicaux $R^4$ et $R^5$ représente un hydrogène et l'autre un nitro ou un cyano, et ou bien $R^2$ représente un hydrogène et $R^3$ un hydrogène ou un alcoyle en $C_1$ à $C_7$, ou bien $R^2$ et $R^3$ représentent ensemble un diméthylène, un triméthylène ou un propénylène et l'atome de carbone désigné par γ présente la configuration (S) ou (R,S), et X représente un atome d'oxygène ou de soufre, et leurs sels d'addition d'acides pharmaceutiquement acceptables.

2. Composé selon la revendication 1, caractérisés en ce que $R^3$ représente un méthyle lorsque $R^2$ représente un hydrogène.

3. Composés selon la revendication 1, caractérisés en ce que l'atome de carbone désigné par γ dans la formule I présente la configuration (S) lorsque $R^2$ et $R^3$ représentent ensemble un diméthylène ou un triméthylène.

4. Ethyl-5,6-dihydro-5-methyl-7-nitro-6-oxo-4H-ímidazo [1,5-a] [1,4] benzodiazépine-3-carboxylate.

5. Ethyl-7-cyano-5,6-dihydro-5-méthyle-6-oxo-4H-imidazo [1,5-a] [1,4] benzodiazépine-3-carboxylate.

6. Ethyl-5,6-dihydro-5-méthyl-8-nitro-6-oxo-4H-imidazo [1,5-a] [1,4] benzodiazépine-3-carboxylate, Ethyl-8-cyano-5,6-dihydro-5-méthyl-5-méthyl-6-oxo-4H-imidazo [1,5-a] [1,4]-benzodiazépine-3-carboxylate et Ethyl-(S)-(+)-11,12,13,13a-tétrahydro-7-nitro-9-oxo-9H-imidazo [1,5-a] pyrrolo [2,1-c] [1,4] benzodiazépine-1-carboxylate.

7. Composés de formule générale II

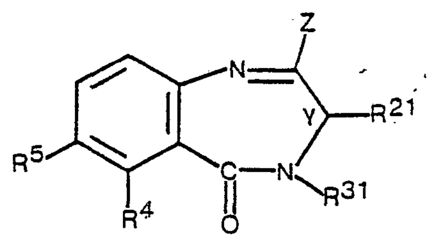

(II)

ou bien $R^{21}$ représente un hydrogène et $R^{31}$ un alcoyle en $C_1$ à $C_7$, ou $R^{21}$ et $R^{31}$ représentent ensemble un diméthylène, un triméthylène ou un propénylène et l'atome de carbone désigné par γ présente la configuration (S) ou (R,S), l'un des radicaux $R^4$ et $R^5$ représente un hydrogène et l'autre un nitro ou un cyano et Z représente un groupe sortant, avec la précision que $R^4$ ou selon les cas $R^5$ ne représente pas un nitro lorsque $R^{21}$ représente un hydrogène et $R^{31}$ représente un alcoyle en $C_1$ à $C_7$.

8. Composés de formule générale

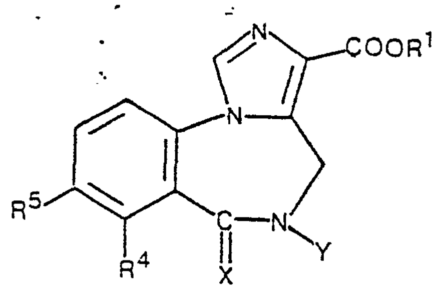

(V)

où $R^1$ représente un méthyle ou un isopropyle, l'un des radicaux $R^4$ et $R^5$ représente un hydrogène et l'autre un nitro ou un cyano, X représente un atome d'oxygène ou de soufre et Y représente un groupe protecteur.

9. Composés de formule générale

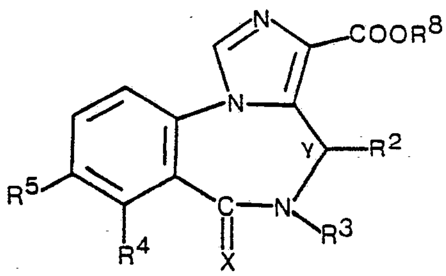

(VII)

où l'un des radicaux $R^4$ et $R^5$ représente un hydrogène et l'autre un nitro ou un cyano, et ou bien $R^2$ représente un hydrogène et $R^3$ un hydrogène ou un alcoyle en $C_1$ à $C_7$, ou bien $R^2$ et $R^3$ représentent ensemble un diméthylène, un triméthylène ou un propénylène et l'atome de carbone désigné par $\gamma$ présente la configuration (S) ou (R,S), X représente un atome d'oxygène ou de soufre et $R^8$ représente un alcoyle en $C_1$ à $C_7$, mais pas un méthyle, un éthyle ni un isopropyle.

10. Composés selon l'une des revendications 1 à 6 comme substances actives pharmaceutiques.

11. Composés selon l'une des revendications 1 à 6 comme substances actives qui ont un rôle d'antagonisme des propriétés sédatives sur le système nerveux central, de relaxation musculaire, atactiques, d'abaissement de la tension artérielle et de dépression de la respiration des 1,4-benzodiazépines à action tranquillisante.

12. Procédé de préparation de composés selon l'une des revendications 1 à 6, caractérisé en ce que

a) on fait réagir un composé de formule générale

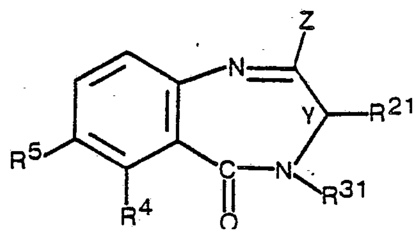

(II)

où $R^4$ et $R^5$ ont la signification donnée dans la revendication 1 et Z représente un groupe sortant, et ou bien $R^{21}$ représente un hydrogène et $R^{31}$ représente un alcoyle en $C_1$ à $C_7$, ou bien $R^{21}$ et $R^{31}$ représente ensemble un diméthylène, un triméthylène ou un propénylène et l'atome de carbone désigné par $\gamma$ présente la configuration (S) ou (R,S), en présence d'une base avec un ester d'acide isocyanacétique de formule générale

$$CN{-}CH_2{-}COOR^1 \qquad (III)$$

où $R^1$ a la signification donnée dans la revendication 1, ou

b) dans un composé de formule générale

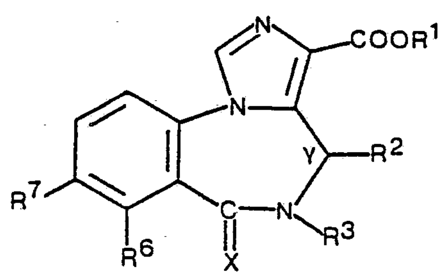

(IV)

où l'un des radicaux $R^6$ et $R^7$ représente un hydrogène et l'autre un halogène, et $R^1$, $R^2$, $R^3$ et X ont la signification donnée dans la revendication 1, on remplace l'atome d'halogène par le groupe cyano, ou

c) on substitue de façon correspondante sur le groupe amino secondaire un composé de formule générale

(Voir Figure page suivante)

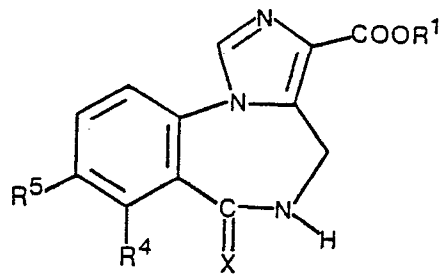

(Ia)

où $R^1$, $R^4$, $R^5$ et X ont la signification donnée dans la revendication 1, ou

d) dans un composé de formule générale

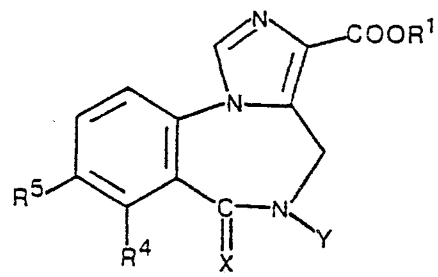

(V)

où $R^1$, $R^4$, $R^5$ et X ont la signification donnée dans la revendication 1, et Y représente un groupe protecteur, on sépare le groupe protecteur, ou

e) on nitre un composé de formule générale

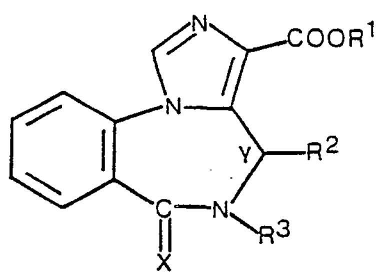

(VI)

où $R^1$, $R^2$, $R^3$ et X ont la signification donnée dans la revendication 1, ou

f) on trans-estérifie un composé de formule générale

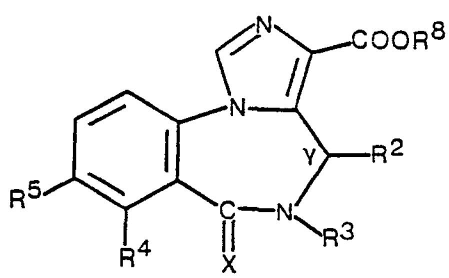

(VII)

où $R^2$, $R^3$, $R^4$, $R^5$ et X ont la signification donnée dans la revendication 1, et $R^8$ représente un alcoyle en $C_1$ à $C_7$, ou

g) dans un composé de formule générale

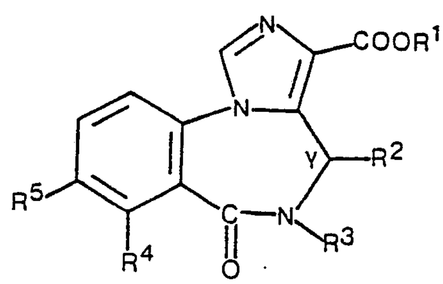

(Ib)

où $R^1$, $R^2$, $R^3$, $R^4$ et $R^5$ ont la signification donnée dans la revendication 1, on transforme le groupe carbonyle en le groupe thiocarbonyle, et

h) si on le désire, on transforme un composé de formule générale I obtenu en un sel d'addition d'acide pharmaceutiquement acceptable.

13. Médicament contenant un composé selon l'une des revendications 1 à 6.

14. Médicament selon la revendication 13 jouant un rôle d'antagoniste des propriétés sédatives du système nerveux central, de relaxation musculaire, atactiques, d'abaissement de la tension artérielle et de dépression de la respiration des 1,4-benzodiazépines à action tranquillisante.

15. Médicament à action schistosomicide contenant un composé selon l'une des revendications 1 à 6 et la (+)-5-(o-chlorophényl)-1,3-dihydro-3-méthyl-7-nitro-2H-1,4-benzodiazépin-2-one.